# EUROPEAN PATENT APPLICATION

(11) **EP 4 371 984 A1**
(43) Date of publication of application: **22.05.2024**
(21) Application number: 22856211.2
(22) Date of filing: 10.08.2022
(51) Int. Cl.: C07D 403/14, A61K 31/409

(54) **METHOD FOR SYNTHESIZING BILIRUBIN**

(30) Priority: 11.08.2021 KR 20210106108
(71) Applicant: Bilix Co., Ltd., Seoul 06241 (KR)
(72) Inventor: KIM, Myung Lip, Busan 48272 (KR); MA, Sang Ho, Suwon-si, Gyeonggi-do 16438 (KR); PARK, Ki Soo, Suwon-si, Gyeonggi-do 16222 (KR); JUN, Hee Goo, Suwon-si, Gyeonggi-do 16508 (KR); KIM, Da Eun, Yongin-si, Gyeonggi-do 16944 (KR)
(74) Representative: BCKIP Part mbB
(86) International application number: PCT/KR2022/011910
(87) International publication number: WO 2023/018214

(57) **Abstract**

The present invention relates to a novel synthesis method of bilirubin. The method includes preparing a compound represented by Formula 2 by dimerizing a compound represented by Formula 1 or by coupling a compound represented by Formula 3 and a compound represented by Formula 4, thereby firstly chemically synthesizing bilirubin and PEGylated bilirubin, which are usefully used in a medical product or the like.

## Description

### [Technical Field]

The present invention relates to a novel synthesis method of bilirubin.

### [Background Art]

Bilirubin is a component of bile and is mostly generated from hemoglobin in the body. Bilirubin is a yellowish final metabolite formed from heme, and despite many hydrophilic groups, it is extremely hydrophobic due to intramolecular hydrogen bonding.

Bilirubin was considered an undesirable substance as it caused jaundice when the blood level was high. However, in a recently published study, it was found that a slightly higher blood concentration of bilirubin significantly lowered the possibility of developing cardiovascular disease or cancer. Further, tissue-protecting effects of bilirubin were confirmed through animal experiments since it functions to remove different active oxygen and control immunocytes related to inflammation.

Although bilirubin is an industrially useful substance, it has been obtained by extracting from animals and has never been successfully synthesized. When bilirubin is extracted from animals, it is difficult to obtain bilirubin in large quantities while causing high production costs. Further, since bilirubin extracted from animals is a mixture of three regioisomers, it should undergo an additional separation and purification process in order to be utilized as a medicine. It is urgent to develop a method capable of chemically producing bilirubin.

### [Summary of Invention]

### [Problems to be Solved by Invention]

An object of the present invention is to provide a method for synthesizing bilirubin.

### [Means for Solving Problems]

1. A method for synthesizing bilirubin, including preparing a compound represented by Formula 2 below by dimerizing a compound represented by Formula 1 below:

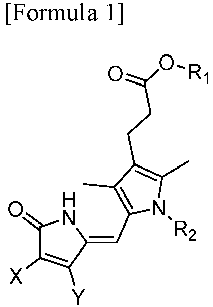

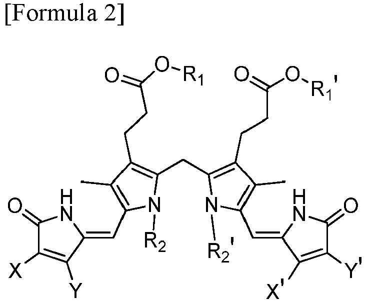

   (wherein, in the above formulas 1 and 2, R₁ and R₁' are each independently hydrogen, an alkyl group having 1 to 12 carbon atoms, an aryl group having 6 to 20 carbon atoms, a heteroaryl group having 2 to 20 carbon atoms, an arylalkyl group having 7 to 20 carbon atoms, or a heteroarylalkyl group having 3 to 20 carbon atoms; R₂ and R₂' are each hydrogen or a nitrogen protecting group; any one of X and Y is a vinyl or acetyl group, or a halogen atom, or an ethyl group substituted with a hydroxyl group, sulfide, selenide or a halogen atom while the other is a methyl group; and any one of X' and Y' is a vinyl or acetyl group, or a halogen atom, or an ethyl group substituted with a hydroxyl group, sulfide, selenide or a halogen atom while the other is a methyl group).
2. The method according to the above 1, further including reacting the compound represented by Formula 2 with polyethylene glycol (PEG).
3. The method according to the above 1, wherein the compound represented by Formula 1 is reacted with polyethylene glycol (PEG) and then dimerized.
4. A compound represented by Formula 3 below:

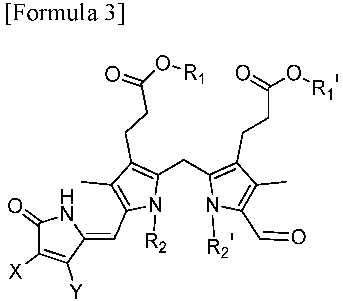

   (wherein, R₁ and R₁' are each independently hydrogen, an alkyl group having 1 to 12 carbon atoms, an aryl group having 6 to 20 carbon atoms, a heteroaryl group having 2 to 20 carbon atoms, an arylalkyl group having 7 to 20 carbon atoms, or a heteroarylalkyl group having 3 to 20 carbon atoms; R₂ and R₂' are each hydrogen or a nitrogen protecting group; and any one of X and Y is a vinyl or acetyl group, or a halogen atom, or an ethyl group substituted with a hydroxyl group, sulfide, selenide or a halogen atom while the other is a methyl group).
5. A method for synthesizing bilirubin, including preparing a compound represented by Formula 2 below by coupling a compound represented by Formula 4 below with the compound represented by Formula 3 of the above 4: 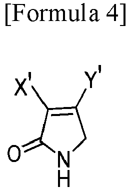
6. The method according to the above 5, further including reacting the compound represented by Formula 2 with polyethylene glycol (PEG).
7. The method according to the above 5, wherein the compound represented by Formula 3 is reacted with polyethylene glycol (PEG) and then coupled with the compound represented by Formula 4.
8. The method according to the above 5, further including reacting a compound represented by Formula 1 below with a compound represented by Formula 5 below to prepare the compound represented by Formula 3: 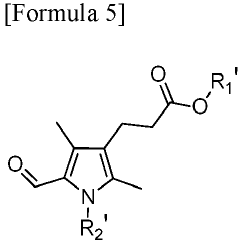 (wherein, R₁, R₁', R₂, R₂', X and Y in Formulas 1 and 5 are the same as R₁, R₁', R₂, R₂', X and Y in Formula 3, respectively).
9. The method according to the above 5, further including substituting a carboxyl group bonded to a pyrrole group of a compound represented by Formula 6 below with an aldehyde group to prepare the compound represented by Formula 3:

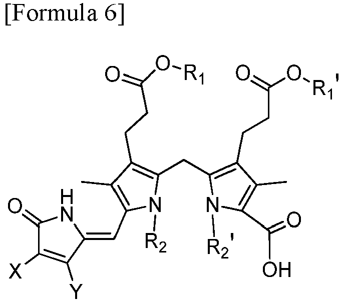

   (wherein, R₁, R₁', R₂, R₂', X and Y are the same as R₁, R₁', R₂, R₂', X and Y in Formula 3, respectively).
10. The method according to the above 9, further including coupling a compound represented by Formula 7 below with the compound represented by Formula 4 of the above 5 to prepare the compound represented by Formula 6:

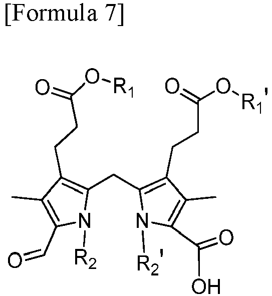

   (wherein, R₁, R₁', R₂ and R₂' are the same as R₁, R₁', R₂ and R₂' in Formula 6, respectively).
11. The method according to the above 5, further including coupling a compound represented by Formula 8 below with the compound represented by Formula 4 to prepare the compound represented by Formula 3:

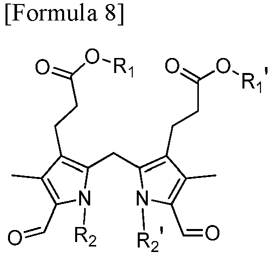

   (wherein, R₁, R₁', R₂ and R₂' are the same as R₁, R₁', R₂ and R₂' in Formula 3, respectively).
12. The method according to the above 10, further including substituting any one carboxyl group bonded to a pyrrole group of a compound represented by Formula 9 below with an aldehyde group to prepare the compound represented by Formula 7:

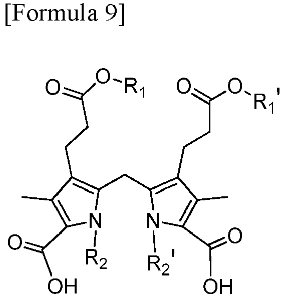

   (wherein, R₁, R₁', R₂ and R₂' are the same as R₁, R₁', R₂ and R₂' in Formula 7, respectively).
13. The method for synthesizing bilirubin according to the above 11, further including substituting a carboxyl group bonded to a pyrrole group of a compound represented by Formula 7 or 9 below with an aldehyde group to prepare the compound represented by Formula 8.

### [Advantageous effects]

The method for synthesizing bilirubin of the present invention may be economically performed under mild conditions.

The method for synthesizing bilirubin of the present invention has a high yield and is suitable for mass production.

### [Brief Description of Drawings]

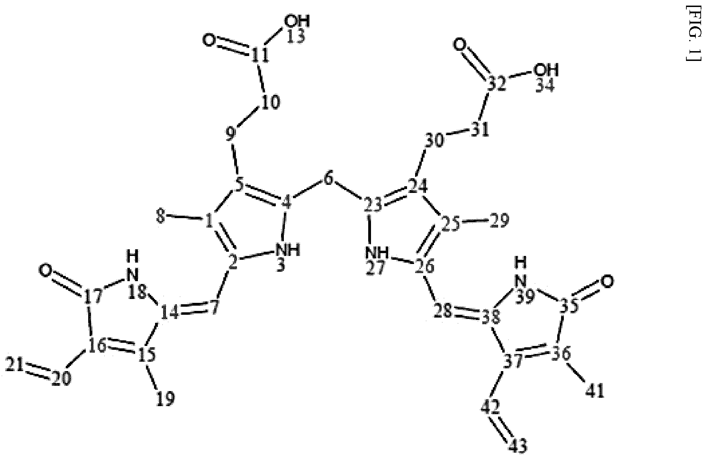

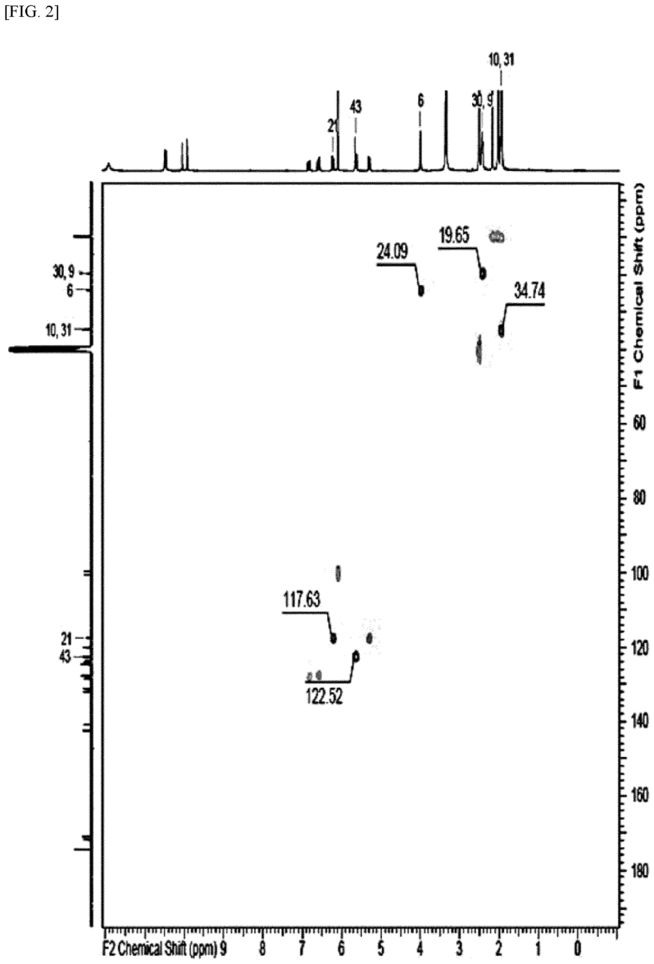

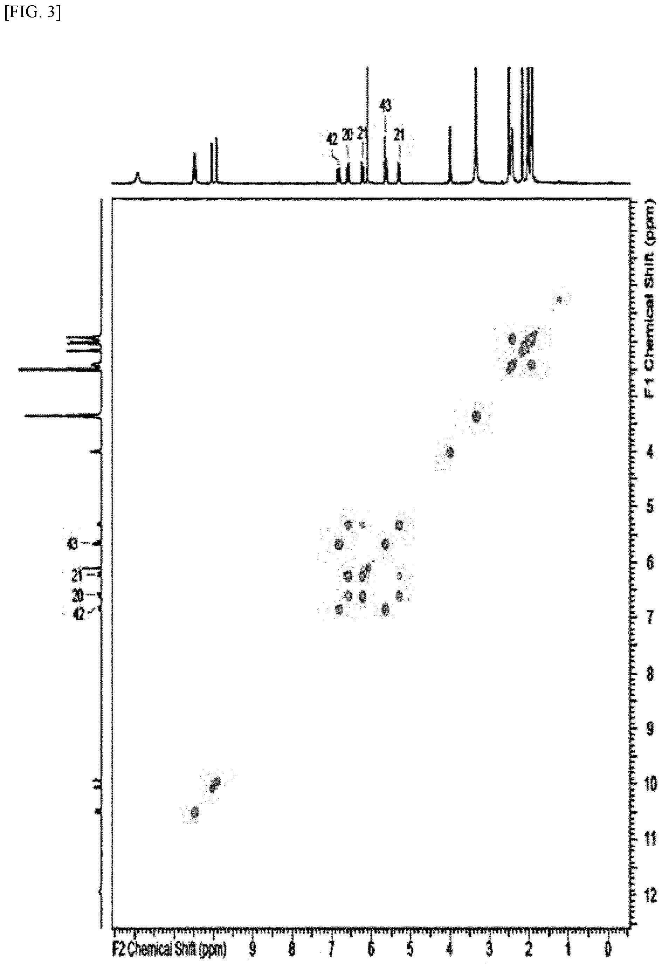

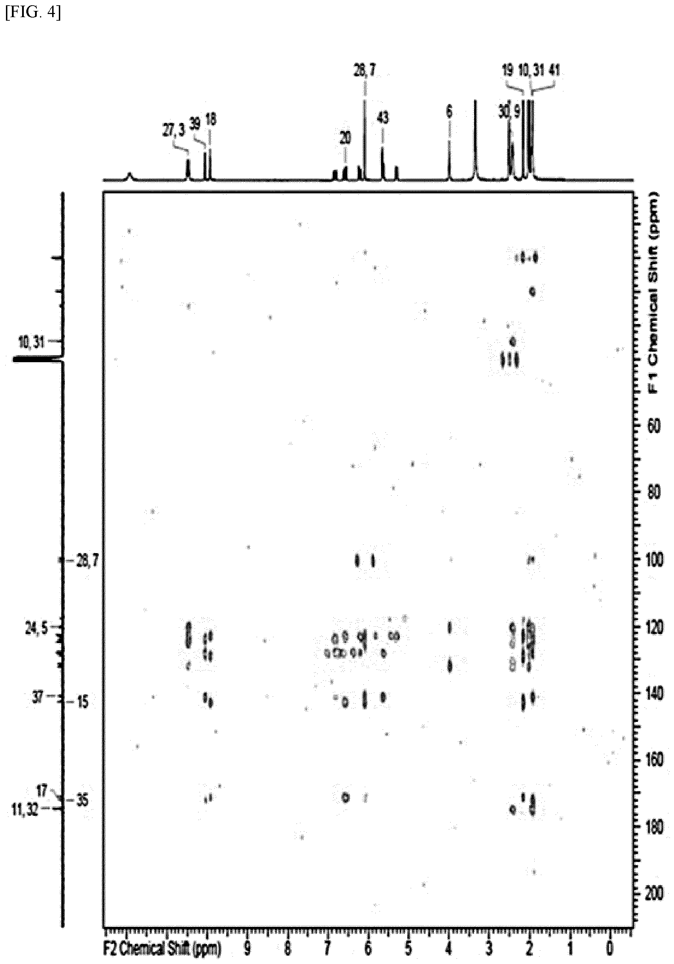

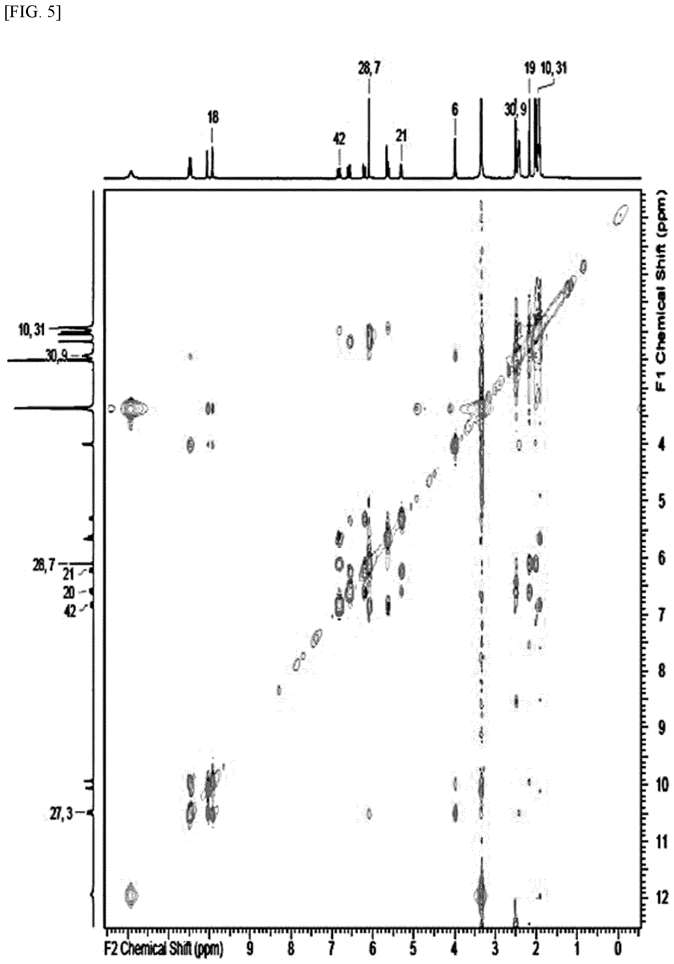

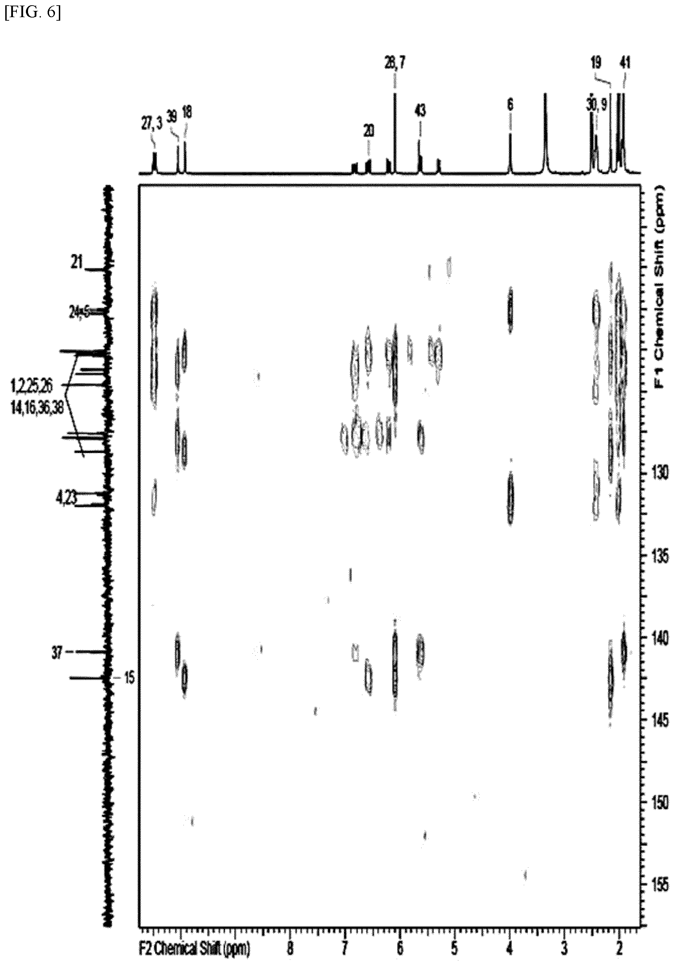

FIGS. 1 to 6 are 2D NMR data of Compound F-9a prepared in Example 19, wherein FIG. 2 is HSQC; FIG. 3 is COSY; FIG. 4 is HMBC; FIG. 5 is NOESY; and FIG. 6 is HMBC data.

### [Mode for Carrying out Invention]

The present invention relates to a novel method for synthesizing bilirubin.

As used herein, the term "alkyl" is a straight or branched, substituted or unsubstituted chain hydrocarbon, such as methyl, ethyl, n-propyl, isopropyl, cyclopropyl, n-butyl, sec-butyl, tert-butyl, cyclobutyl, cyclopropylmethyl, n-pentyl, isopentyl, neopentyl, tert-pentyl, cyclopentyl, cyclobutylmethyl, n-hexyl, isohexyl, cyclohexyl, cyclopentylmethyl and the like.

The term "cycloalkyl" is a monocyclic or bicyclic, substituted or unsubstituted cyclic hydrocarbon, such as cyclopropyl, cyclobutyl, cyclopentyl, cyclohexyl, cycloheptyl, cyclooctyl and the like.

The term "heterocycloalkyl" is a monocyclic or bicyclic, substituted or unsubstituted cyclic hydrocarbon containing one or more heteroatoms selected from B, N, O, S, P(=O), Si and P, such as tetrahydropyranyl, azetidyl, 1,4-dioxanyl, piperazinyl, piperidinyl, pyrrolidinyl, morpholinyl, thiomorpholinyl, dihydrofuranyl, dihydroimidazolyl, dihydroindolyl dihydroisooxazolyl, dihydroisothiazolyl, dihydrooxadiazolyl, dihydrooxazolyl, dihydropyrazinyl, dihydropyrazolyl, dihydropyridyl, dihydropyrimidinyl, dihydropyrrolyl, dihydroquinolyl, dihydrotetrazolyl, dihydrothiadiazolyl, dihydrothiazolyl, dihydrothienyl, dihydrotriazolyl, dihydro-azetidyl, methylenedioxybenzoyl, tetrahydrofuranyl, tetrahydrothienyl and the like.

The term "aryl" is a monocyclic or bicyclic, substituted or unsubstituted aromatic group, such as phenyl, biphenyl, terphenyl, naphthyl, binapthyl, phenylnaphthyl, naphthylphenyl, phenylterphenyl, fluorenyl, phenylfluorenyl, diphenylfluorenyl, benzofluorenyl, dibenzofluorenyl, phenanthrenyl, phenylphenanthrenyl, anthracenyl, indenyl, triphenylenyl, pyrenyl, tetracenyl, perylenyl, chrysenyl, naphthacenyl, fluoranthenyl, spirobifluorenyl, azyurenyl and the like.

"Aryl" includes, for example, phenyl, 1-naphthyl, 2-naphthyl, 1-anthryl, 2-anthryl, 9-anthryl, benzanthryl, 1-phenanthryl, 2-phenanthryl, 3-phenanthryl, 4-phenanthryl, 9-phenanthryl, naphthacetyl, pyrenyl, 1-chrysenyl, 2-chrysenyl, 3-chrysenyl, 4-chrysenyl, 5-chrysenyl, 6-chrysenyl, benzo[c]phenanthryl, benzo[g]chrysenyl, 1-triphenylenyl, 2-triphenylenyl, 3-triphenylenyl, 4-triphenylenyl, 1-fluorenyl, 2-fluorenyl, 3-fluorenyl, 4-fluorenyl, 9-fluorenyl, benzofluorenyl, dibenzofluorenyl, 2-biphenylyl, 3-biphenylyl, 4-biphenylyl, o-terphenyl, m-terphenyl-4-yl, m-terphenyl-3-yl, m-terphenyl-2-yl, p-terphenyl-4-yl, p-terphenyl-3-yl, p-terphenyl-2-yl, m-quaterphenyl, 3-fluoranthenyl, 4-fluoranthenyl, 8-fluoranthenyl, 9-fluoranthenyl, benzofluoranthenyl, o-tolyl, m-tolyl, p-tolyl, 2,3-xylyl, 3,4-xylyl, 2,5-xylyl, mesityl, o-cumenyl, m-cumenyl, p-cumenyl, p-tert-butylphenyl, p-(2-phenylpropyl)phenyl, 4'-methylbiphenylyl, 4"-tert-butyl-p-terphenyl-4-yl, 9,9-dimethyl-1-fluorenyl, 9,9-dimethyl-2-fluorenyl, 9,9-dimethyl-3-fluorenyl, 9,9-dimethyl-4-fluorenyl, 9,9-diphenyl-1-fluorenyl, 9,9-diphenyl-2-fluorenyl, 9,9-diphenyl-3-fluorenyl, 9,9-diphenyl-4-fluorenyl and the like.

The term "heteroaryl" refers to a monocyclic or bicyclic, substituted or unsubstituted aromatic group containing one or more heteroatoms selected from B, N, O, S, P(=O), Si and P, such as benzothienyl, benzoxazolyl, benzofuranyl, benzimidazolyl, benzthiazolyl, benzotriazolyl, sinnolinyl, furyl, imidazolyl, tetrazolyl, indazolyl, indolyl, isoxazolyl, isoquinolinyl, isothiazolyl, naphthyridinyl, oxadiazolyl, oxazolyl, isoxazolyl, purinyl, thiazolyl, isothiazolyl, thienopyridinyl, thienyl, thiadiazolyl, pyridinyl, pyridazinyl, pyrimidinyl, pyrazinyl, pyrazolyl, pyrrolyl, pyrido[2,3-d]pyrimidinyl, pyrrolo[2,3-b]pyridinyl, quinazolinyl, quinolinyl, thieno[2,3-c]pyridinyl, triazinyl and the like.

The term "arylalkyl" refers to an alkyl group in which at least one of the substituents is substituted with aryl, while "aryl" and "alkyl" are as defined above. For example, this includes benzyl, phenylethyl, phenylpropyl, phenylbutyl, phenylhexyl, naphthylethyl, naphthylpropyl, naphthylbutyl, naphthylhexyl, anthracenylmethyl, anthracenylethyl, anthracenylpropyl, anthracenylbutyl, phenanthrylmethyl, phenanthryl ethyl, phenanthrylpropyl, triphenylmethyl, triphenylethyl, triphenylpropyl, pyrenylmethyl, pyrenylethyl, pyrenylpropyl, phenylanthracenemethyl, phenylanthraceneethyl, phenylanthracenepropyl, perylenylmethyl, perylenylethyl, perylenylpropyl, chrysenylmethyl, chrysenylethyl, chrysenylpropyl, fluorenylmethyl, fluorenylethyl, fluorenylpropyl and the like.

The term "heteroarylalkyl" refers to an alkyl group in which at least one of the substituents is substituted with heteroaryl, while heteroaryl and alkyl are as defined above. For example, this includes pyridinylmethyl, pyridinylethyl, pyridinylpropyl, pyridinylbutyl, pyrimidinylmethyl, pyrimidinylethyl, pyrimidinylpropyl, pyrazolylmethyl, pyrazolylethyl, pyrazolylmethyl, pyrazolylethyl, pyrazolylpropyl, quinolinylmethyl, quinolinylethyl, quinolinylpropyl and the like.

The term "substituted" refers to inclusion of at least one substituent, for example, one or two or more of halogen atom, nitro, hydroxyl, cyano, amino, thiol, carboxyl, amide, nitrile, sulfide, disulfide, sulfenyl, formyl, formyloxy, formylamino, aryl or substituted aryl.

In the case where no substituent is described in a formula of the present invention even though it is a site requiring a substituent, it is considered that a hydrogen substituent is omitted.

The present invention provides a method for synthesizing bilirubin, which includes a step of dimerizing a compound represented by Formula 1 to prepare a compound represented by Formula 2.

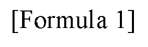

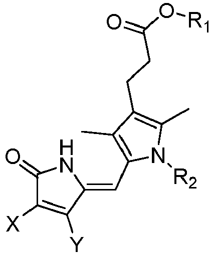

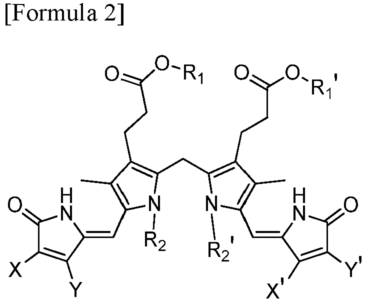

Wherein, in Formulas 1, 2 and 3, R₁ and R₂ are each independently hydrogen, an alkyl group having 1 to 12 carbon atoms, an aryl group having 6 to 20 carbon atoms, a heteroaryl group having 2 to 20 carbon atoms, an arylalkyl group having 7 to 20 carbon atoms, or a heteroarylalkyl group having 3 to 20 carbon atoms.

For example, the number of carbon atoms in R₁ and R₁' may be appropriately selected within a range that does not affect dimerization reaction of the compound represented by Formula 1.

For example, R₁ and R₁' may be each independently an alkyl group having 1 to 10 carbon atoms, an aryl group having 6 to 10 carbon atoms, a heteroaryl group having 2 to 10 carbon atoms, an arylalkyl group having 7 to 10 carbon atoms, or a heteroarylalkyl group having 3 to 10 carbon atoms.

Further, R₁ and R₁' may be each independently an alkyl group having 1 to 5 carbon atoms, an aryl group having 6 to 10 carbon atoms, a heteroaryl group having 4 to 10 carbon atoms, an arylalkyl group having 7 to 10 carbon atoms, or a heteroarylalkyl group having 5 to 10 carbon atoms.

R₂ and R₂' are each hydrogen or a nitrogen protecting group.

Herein, the nitrogen-protecting group is not limited to a specific one as long as it is a substituent capable of protecting the nitrogen atom to which R₄ is bonded and may be selected from the group consisting of, for example, -COORₓ (Rₓ is as defined above), tert-butyloxycarbonyl (Boc), trityl (-CPh₃), tosyl group (SOOPhCH₃), 9-fluorenylmethyloxycarbonyl (Fmoc), carboxybenzyl group (Cbz), p-methoxybenzyl carbonyl (Moz), acetyl (Ac), benzoyl (Bz), p-methoxybenzyl (PMB), 3,4-dimethoxybenzyl (DMPM), p-methoxyphenyl (PMP), 2-naphthylmethyl ether (Nap), and trichloroethyl chloroformate (Troc).

When the nitrogen-protecting group of R₂ and/or R₂' of Formula 2 remains after dimerization of the compound represented by Formula 1, a step of removing the nitrogen-protecting group may be additionally required.

Any one of X and Y is a vinyl or acetyl group, or a halogen atom, or an ethyl group substituted with a hydroxyl group, sulfide, selenide or a halogen atom while the other is a methyl group. For example, X may be a vinyl group and Y may be a methyl group, or X may be an ethyl group substituted with a hydroxyl group while Y may be a methyl group.

Any one of X' and Y' is a vinyl or acetyl group, or a halogen atom, or an ethyl group substituted with a hydroxyl group, sulfide, selenide or a halogen atom while the other is a methyl group. For example, X' may be a vinyl group and Y' may be a methyl group, or X' may be an ethyl group substituted with a hydroxyl group while Y' may be a methyl group.

Here, selenide is a functional group having a structure of Formula 11 and sulfide is a functional group having a structure of Formula 12.

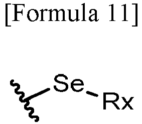

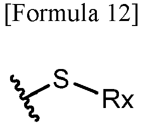

In Formulas 11 and 12, Rx may be hydrogen, or a substituted or unsubstituted, straight-chain or branched alkyl, cycloalkyl, heterocycloalkyl, aryl, heteroaryl, arylalkyl or heteroarylalkyl group.

For example, Rx is an alkyl group having 1 to 12 carbon atoms, a cycloalkyl group having 5 to 20 carbon atoms, a heterocycloalkyl group having 2 to 20 carbon atoms, an aryl group having 6 to 20 carbon atoms, a heteroaryl group having 2 to 20 carbon atoms, an arylalkyl group having 7 to 20 carbon atoms or a heteroarylalkyl group having 3 to 20 carbon atoms.

For example, R_{X} is a phenyl or p-tolyl group.

R₃ may be an ethyl group substituted with a hydroxyl group. For example, it is a functional group in which a hydroxyl group is substituted at the position of carbon No. 1 of the ethyl group.

R₃ may be an ethyl group substituted with selenide. For example, it is a functional group in which selenide is substituted at the position of carbon No. 2 of the ethyl group.

R₃ may be an ethyl group substituted with sulfide. For example, it is a functional group in which sulfide is substituted at the position of carbon No. 2 of the ethyl group.

The dimerization reaction of the compound represented by Formula 1 may be, for example, a coupling between the compounds of Formula 1 wherein R₁ = hydrogen, R₂ = hydrogen, X = a vinyl group and Y = a methyl group; or a coupling between the compound of Formula 1 wherein R₁ = hydrogen, R₂ = hydrogen, X = a vinyl group and Y = a methyl group, and the compound of Formula 1 wherein R₁ = hydrogen, R₂ = hydrogen, X = a methyl group and Y = a vinyl group.

The dimerization reaction of the compound represented by Formula 1 is shown in Schemes 1 to 3 below.

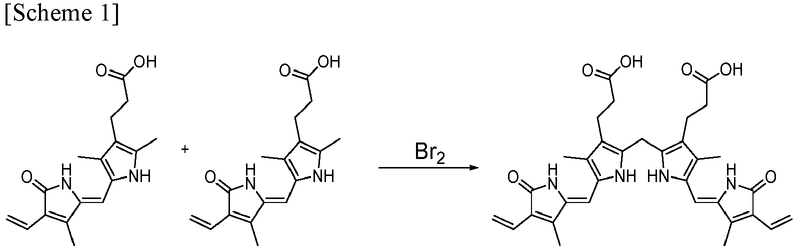

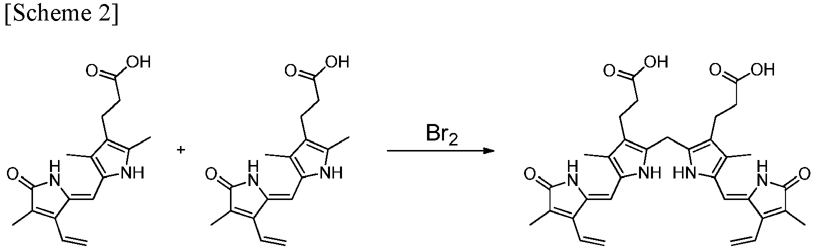

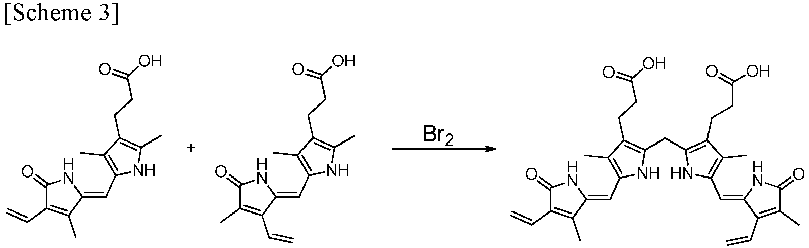

The dimerization reaction may be carried out, for example, under bromine or chloranil condition.

The dimerization reaction may be carried out under a solvent.

The solvent is an inorganic solvent or an organic solvent. The organic solvent may be, for example, alcohols, ethers, ketones, aliphatic hydrocarbons, aromatic hydrocarbons, halogenated hydrocarbons, alkoxies, nitriles or amides. Solvents belonging to these classes are, for example, listed in Table 1. The inorganic solvent is, for example, water.

**[TABLE 1]**

| Division | Organic solvent |
|---|---|
| Alcohols | Methanol, ethanol, propanol, isopropanol, ethylene glycol |
| Ethers | Diethylether, tetrahydrofurane (THF), 2-methyl tetrahydrofuran, dioxanes |
| Ketones | Methyl cellosolve, ethyl cellosolve, butyl cellosolve, methylethyl ketone, acetone |
| Aliphatic hydrocarbons | Hexane, heptane, octane |
| Aromatic hydrocarbons | Benzene, toluene, xylene |
| Halogenated hydrocarbons | Dichloromethane (DCM), chloroform, chlorobenzene |
| Alkoxies | Methoxyethane, Dimethoxyethane(DME), methoxypropane, dimethoxypropane |
| Nitriles | Acetonitrile, benzonitrile, trinitrile |

The dimerization reaction may be performed at 10 to 100 °C, for example, 10 to 80 °C, 20 to 60 °C, 20 to 50 °C, or 20 to 30 °C. The optimal reaction temperature may vary depending on the solvent used.

The dimerization reaction may be performed for 1 to 24 hours, for example, for 1 to 18 hours or 1 to 12 hours.

The method for synthesizing bilirubin of the present invention may further include converting R₁ and/or R₁' of the compound represented by Formula 2 into hydrogen through a saponification reaction. For example, when R₁ and R₁' of the compound represented by Formula 2 are a methyl group, a base such as LiOH, KOH or NaOH may be added to the compound represented by Formula 2 in order to replace the methyl group with hydrogen.

The solvent used for the saponification reaction is not particularly limited. As the solvent for saponification, the same solvent as for the dimerization reaction may be used. For example, it may be methanol, ethanol, 2-propanol, tetrahydrofuran (THF), 2-methyltetrahydrofuran (2-MeTHF), dioxane, acetonitrile, N,N-dimethylformamide (DMF), t-butanol, dimethoxyethane (DME), dichloromethane (DCM), isopropyl alcohol or the like.

The saponification may be carried out under conditions known in the art. For example, it may be performed at 10 to 150 °C for 1 to 72 hours, or at 10 to 60 °C for 1 to 48 hours.

The method for synthesizing bilirubin of the present invention may further include a PEGylation step of reacting the compound represented by Formula 2 with polyethylene glycol (PEG).

The method for synthesizing bilirubin of the present invention may include a step of PEGylating the compound represented by Formula 1 with polyethylene glycol (PEG) and then dimerizing the resulting product.

PEGylated bilirubin has improved water solubility.

Polyethylene glycol is, for example, mPEGₙ -NH₂ (methoxy polyethylene glycol-amine, n=5 to 60). Herein, n is the number of -CH₂-CH₂-O- repeating units of methoxy polyethylene glycol-amine, which may range from 5 to 60, 10 to 50, 10 to 40, 20 to 40, 10 to 30, or 20 to 30.

PEGylation includes mono-PEGylation in which either O-R₁ or O-R₂ is PEGylated, and bi-PEGylation in which both of them are PEGylated.

In the PEGylation reaction, polyethylene glycol may be added in an appropriate amount in consideration of the number of moles of the compound represented by Formula 1 or Formula 2. For example, polyethylene glycol may be added in an amount of 0.1 to 10 moles, 0.1 to 8 moles, 0.1 to 5 moles, 0.3 to 8 moles, 0.3 to 5 moles, or 0.3 to 4 moles, or 0.3 to 3 moles based on 1 mole of the compound represented by Formula 1 or Formula 2.

As reagents for PEGylation reaction, CDI (1,1-carbonyldiimidazole), CMPI (2-chloro-1-methylpyridinium iodide), BEP (2-bromo-1-ethyl-pyridinium tetrafluoroborate), EDCI (1-ethyl-3-(3-dimethylaminopropyl)carbodiimide), HATU (1-[bis(dimethylamino)methylene]-1H-1,2,3-triazolo[4,5-b]pyridinium 3-oxide hexafluorophosphate; hexafluorophosphate azabenzotriazole tetramethyl uronium, DCC (N,N'-dicyclohexylcarbodiimide) or HOBt (hydroxybenzotriazole), etc. may be used, but it is not limited thereto.

The reagent for PEGylation reaction may be added in an amount of 0.3 to 5 moles, 0.3 to 3 moles, 0.5 to 5 moles, 0.5 to 3 moles, 0.5 to 2.5 moles or 0.5 to 2 moles based on 1 mole of the compound represented by Formula 1 or Formula 2, but it is not limited thereto.

The solvent for PEGylation reaction is not particularly limited. As the solvent for PEGylation reaction, the same solvent as for coupling reaction may be used. For example, it may be DMSO (dimethyl sulfoxide), DMF (dimethylformamide), DMA (dimethylacetamide) or pyridine.

The PEGylation reaction may be carried out in the presence of a base.

The base is an organic base or an inorganic base.

As the organic base, it is preferable to use an amine organic base. For example, it may be chain type amine organic bases such as methylamine, ethylamine, dimethylamine, diethylamine, ethylmethylamine, propylamine, dipropylamine, methylpropylamine, ethylpropylamine, diisopropylamine, N-methylcyclohexylamine or trimethylamine, etc.; or cyclic amine organic bases such as aziridine, azetidine, oxaziridine, azetidine, diazetidine, imidazolidine, pyrazolidine, oxazolidine, isoxazolidine, thiazolidine, isothiazolidine, piperidine, 2-methylpiperidine, 2-ethylpiperidine, 2,6-dimethylpiperidine, N-methylpiperidine, N-ethylpiperidine, 2,6-dimethylpiperidine, 2,2,6,6-tetramethylpiperidine, 3-methylpiperidine, 3-ethylpiperidine, 1-methyl-4-(methylamino)piperidine, 4-aminopiperidine, pyrrolidine, 2-pyrrolidine carboxamide, pyrrolidin-3-ol, piperazine, 2,6-dimethylpiperazine, 1-benzylpiperazine, 1-isopropylpiperazine, 2-ethylpiperazine, N-propylpiperazine, morpholine, thiomorpholine, 4-methyl morpholine, 2,6-dimethyl morpholine, ethyl morpholine, azepane, 2-methyl azepane, 4-methyl azepane, 2,2,7,7-tetramethyl azepane, 1,2,2-trimethyl azepane, 1,2-dimethyl azepane, 2,7-dimethyl azepane, azocane, 1,2-dimethyl azocane, 1,2,2-trimethyl azocane, methyl azocane-2-carboxylate, 1-methyl azocane, 2-(2-methylphenyl) azocane or proline, etc.

A preferred organic base for PEGylation reaction is N,N-Diisopropylethylamine (DIPEA) or pyridine.

The inorganic base may be, for example, LiOH, KOH or NaOH.

The base may be included in an amount of 2 to 20 moles, 2 to 15 moles, 2 to 10 moles, 4 to 20 moles, 4 to 15 moles, 4 to 10 moles, or 5 to 20 moles, 5 to 15 moles, 5 to 10 moles, 6 to 20 moles, 6 to 15 moles or 6 to 10 moles based on 1 mole of the compound represented by Formula 1 or Formula 2.

The PEGylation reaction may be carried out at 10 to 100 °C, such as 10 to 80 °C, 20 to 60 °C, 20 to 50 °C, or 20 to 30 °C.

The PEGylation reaction may be carried out for 1 to 24 hours, 1 to 18 hours, and 1 to 12 hours, but it is not limited thereto.

In one embodiment, the PEGylation reaction may be performed by adding 0.3 to 5 moles of polyethylene glycol and 0.5 to 5 moles of a coupling reagent (CDI, EDCI, CMPI, etc.) based on 1 mole of the compound represented by Formula 1 or Formula 2, and this reaction may be carried out at 20 to 40 °C for 0.5 to 24 hours.

The present invention provides a compound represented by Formula 3. 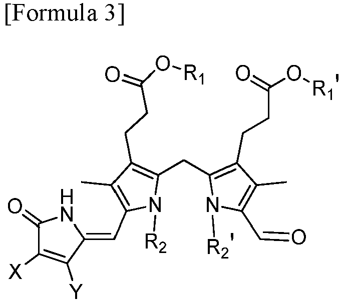 wherein R₁, R₁', R₂, R₂', X and Y are the same as R₁, R₁', R₂, R₂', X and Y in Formula 2, respectively. For example, R₁ and R₁' are each independently hydrogen, an alkyl group having 1 to 12 carbon atoms, an aryl group having 6 to 20 carbon atoms, a heteroaryl group having 2 to 20 carbon atoms, an arylalkyl group having 7 to 20 carbon atoms, or a heteroarylalkyl group having 3 to 20 carbon atoms; R₂ and R₂' are each hydrogen or a nitrogen protecting group; and any one of X and Y is a vinyl or acetyl group, or a halogen atom, or an ethyl group substituted with a hydroxyl group, sulfide, selenide or a halogen atom while the other is a methyl group.

The compound represented by Formula 3 is a novel compound capable of synthesizing bilirubin, and bilirubin may be synthesized by coupling the above compound with the compound represented by Formula 4 herein.

The present invention provides a method for synthesizing bilirubin, which includes a step of coupling a compound represented by Formula 3 and a compound represented by Formula 4. 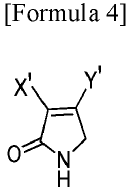 wherein X' and Y' are the same as X' and Y' in Formula 2, respectively.

The coupling reaction is carried out in the presence of a solvent and base.

As the solvent for the coupling reaction, the same solvent as for the dimerization reaction may be used.

As the base for the coupling reaction, it is preferable to use a stronger base than the compound represented by Formula 4.

As the base for the coupling reaction, it is preferable to use an amine organic base. For example, it may be chain type amine organic bases such as methylamine, ethylamine, dimethylamine, diethylamine, ethylmethylamine, propylamine, dipropylamine, methylpropylamine, ethylpropylamine, diisopropylamine, N-methylcyclohexylamine or trimethylamine, etc.; or cyclic amine organic bases such as aziridine, azetidine, oxaziridine, azetidine, diazetidine, imidazolidine, pyrazolidine, oxazolidine, isoxazolidine, thiazolidine, isothiazolidine, piperidine, 2-methylpiperidine, 2-ethylpiperidine, 2,6-dimethylpiperidine, N-methylpiperidine, N-ethylpiperidine, 2,6-dimethylpiperidine, 2,2,6,6-tetramethylpiperidine, 3-methylpiperidine, 3-ethylpiperidine, 1-methyl-4-(methylamino)piperidine, 4-aminopiperidine, pyrrolidine, 2-pyrrolidine carboxamide, pyrrolidin-3-ol, piperazine, 2,6-dimethylpiperazine, 1-benzylpiperazine, 1-isopropylpiperazine, 2-ethylpiperazine, N-propylpiperazine, morpholine, thiomorpholine, 4-methyl morpholine, 2,6-dimethyl morpholine, ethyl morpholine, azepane, 2-methyl azepane, 4-methyl azepane, 2,2,7,7-tetramethyl azepane, 1,2,2-trimethyl azepane, 1,2-dimethyl azepane, 2,7-dimethyl azepane, azocane, 1,2-dimethyl azocane, 1,2,2-trimethyl azocane, methyl azocane-2-carboxylate, 1-methyl azocane, 2-(2-methylphenyl) azocane or proline, etc.

The organic base of coupling reaction is preferably piperidine, pyrrolidine, morpholine, piperazine, azepane, azocane, N-methylpiperidine, N-ethylpiperidine or proline.

The base may be included in an amount of 2 to 20 moles, 2 to 15 moles, 2 to 10 moles, 4 to 20 moles, 4 to 15 moles, 4 to 10 moles, or 5 to 20 moles, 5 to 15 moles, 5 to 10 moles, 6 to 20 moles, 6 to 15 moles or 6 to 10 moles based on 1 mole of the compound represented by Formula 3.

The coupling reaction temperature of the present invention is -20 to 200 °C. For example, it may be 30 to 180 °C, 30 to 150 °C, 30 to 120 °C, 30 to 100 °C, 40 to 150 °C, 40 to 140 °C, 40 to 120 °C, 40 to 100 °C, 50 to 150 °C, 50 to 120 °C, or 50 to 100 °C. The optimum reaction temperature may vary depending on the solvent and base used.

The coupling reaction time of the present invention may be 10 minutes to 120 hours, for example, 1 to 72 hours, 1 to 48 hours, 1 to 24 hours, 3 to 72 hours, 3 to 48 hours, 3 to 24 hours, 6 to 72 hours, 6 to 48 hours, or 6 to 24 hours. The optimal reaction time may vary depending on the solvent and base used.

The method for synthesizing bilirubin of the present invention may further include converting R₁ and/or R₁' of the compound represented by Formula 2 prepared above into hydrogen through a saponification reaction.

The method for synthesizing bilirubin of the present invention may further include a PEGylation step of reacting the compound represented by Formula 2 with polyethylene glycol (PEG).

The method for synthesizing bilirubin of the present invention may include a step of PEGylating the compound represented by Formula 3 with polyethylene glycol (PEG) and then coupling the resulting product with the compound represented by Formula 4.

The PEGylation reaction is carried out under the same solvent and base as the PEGylation reaction described above.

The PEGylation reaction is carried out in the same reaction temperature and time range as the PEGylation reaction described above.

The coupling reaction between the compound represented by Formula 3 and the compound represented by Formula 4 is, for example, shown in Schemes 4 to 10 below.

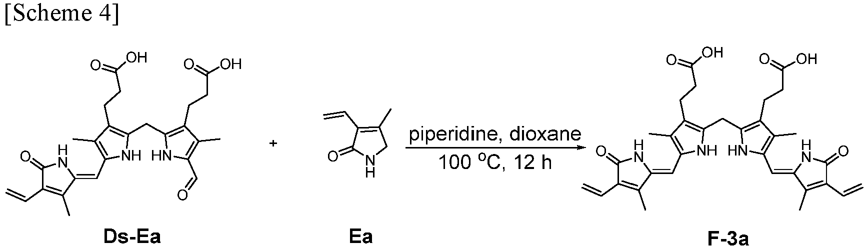

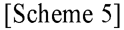

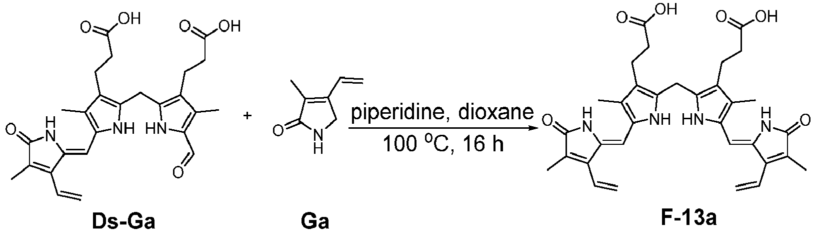

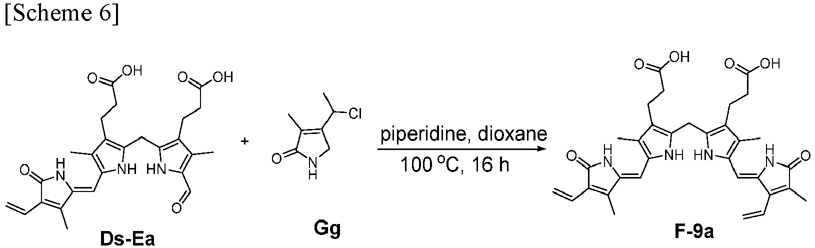

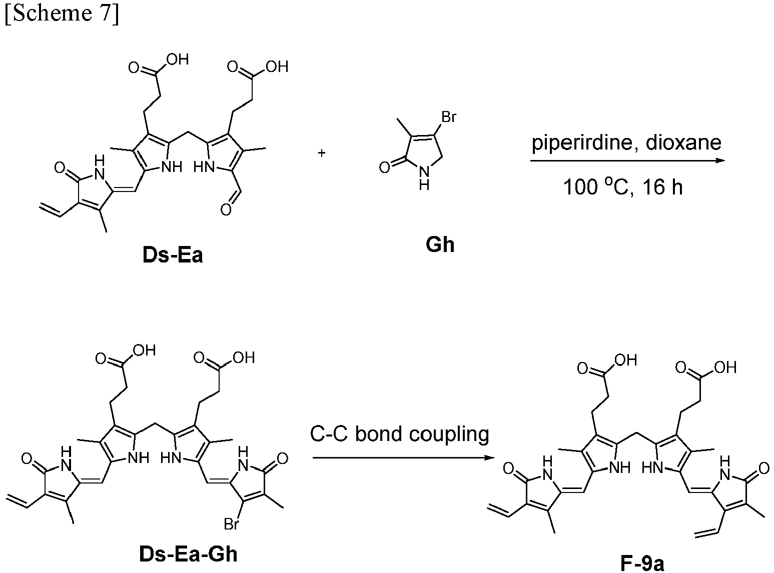

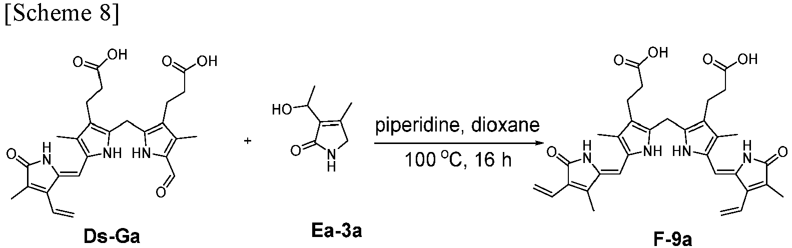

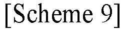

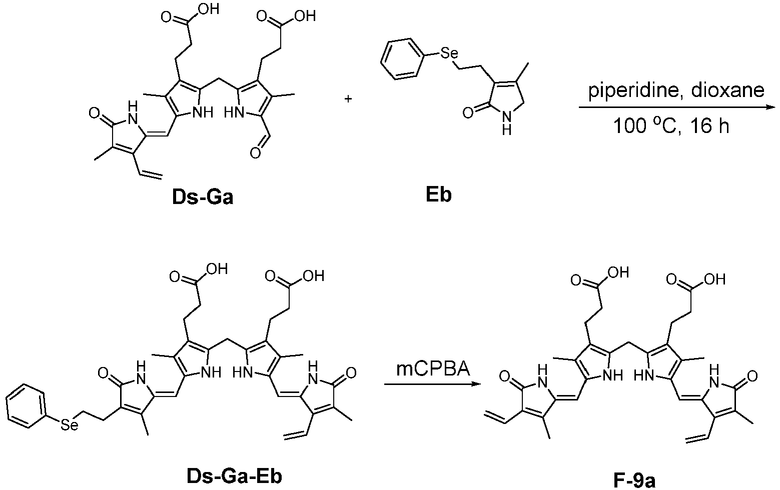

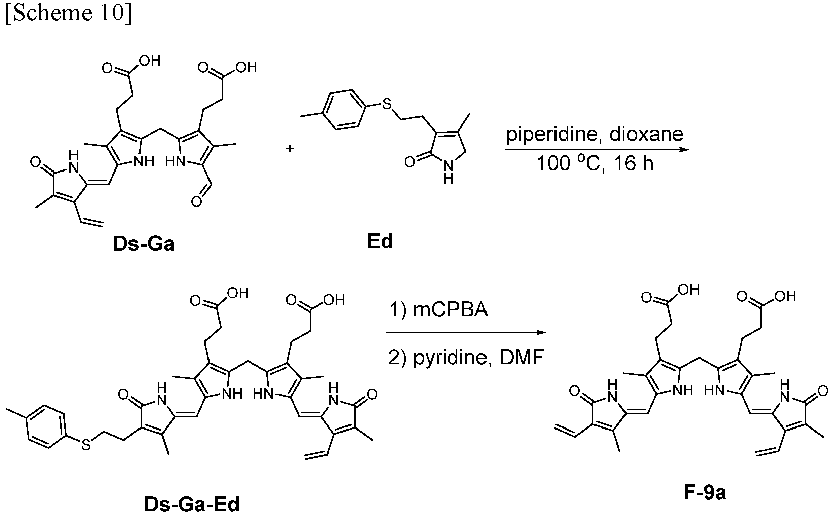

Compound Gg of Scheme 6 may be prepared by the reaction of Scheme 11 below.

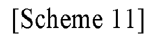

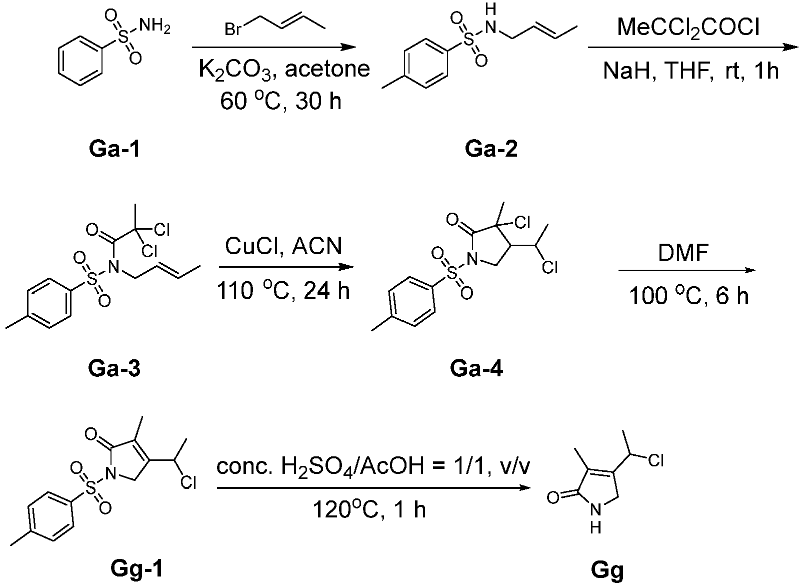

Gh of Scheme 7 may be prepared by the reaction of Scheme 12 below.

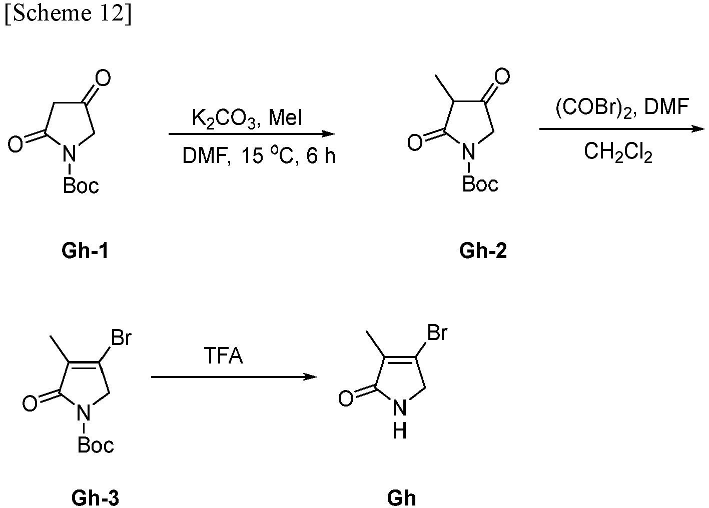

Eb of Scheme 9 may be prepared by the reaction of Scheme 13 below.

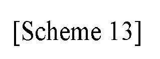

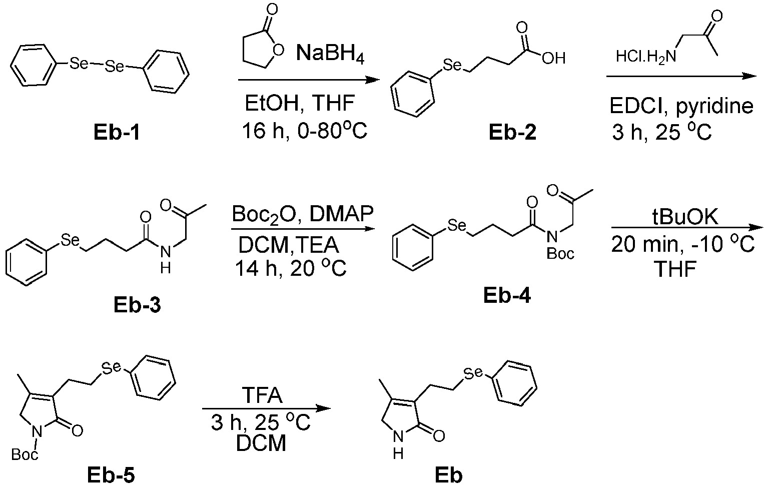

The present invention provides a method for synthesizing bilirubin, which includes a step of reacting the compound represented by Formula 1 with the compound represented by Formula 5 to prepare a compound represented by Formula 3.

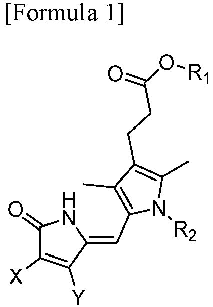

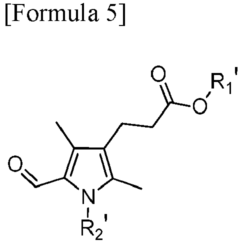

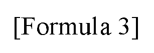

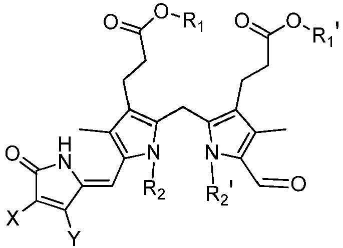

Formula 3 is the same as defined above.

In Formulas 1 and 5, R₁, R₁', R₂, R₂', X and Y are the same as R₁, R₁', R₂, R₂', X and Y in Formula 3, respectively.

The reaction between the compound represented by Formula 1 and the compound represented by Formula 5 may be carried out under bromine condition.

The reaction of the compound represented by Formula 1 and the compound represented by Formula 5 may be carried out under the same condition as the solvent, reaction temperature, and reaction time in the dimerization reaction described above.

The method for synthesizing bilirubin of the present invention may include a step of PEGylating the compound represented by Formula 1 with polyethylene glycol (PEG) and then reacting the resulting product with the compound represented by Formula 5.

The reaction between the compound represented by Formula 1 and the compound represented by Formula 5 is, for example, shown in Schemes 14 and 15 below.

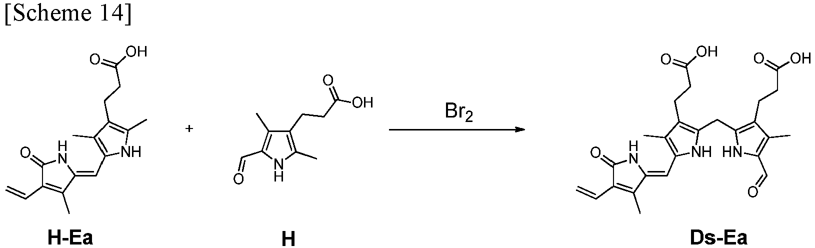

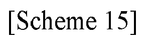

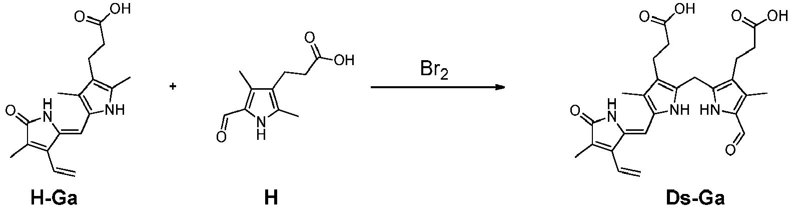

The present invention provides a method for synthesizing bilirubin, which includes a step of substituting a carboxyl group bonded to a pyrrole group of a compound represented by Formula 6 with an aldehyde group to prepare the compound represented by Formula 3.

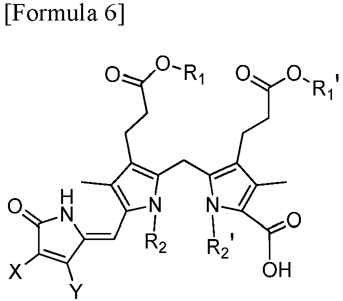

In Formula 6, R₁, R₁', R₂, R₂', X and Y are the same as R₁, R₁', R₂, R₂', X and Y in Formula 3, respectively.

A carboxyl group is reduced by a known method or a carboxyl group is removed and then an aldehyde group is added to substitute the carboxyl group with the aldehyde group. For example, a carboxyl group may be reduced to an aldehyde group under trimethoxymethane and TFA.

Substitution of the carboxyl group bonded to the pyrrole group of the compound represented by Formula 6 with an aldehyde group is, for example, shown in Schemes 16 and 17 below.

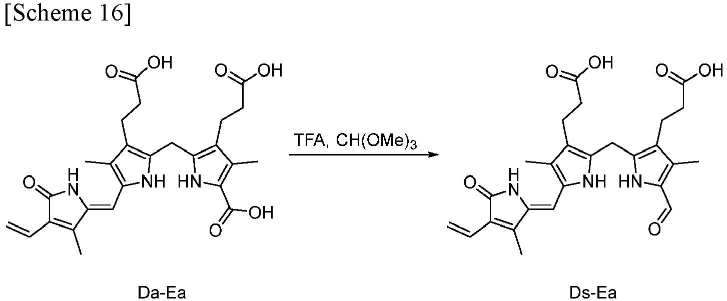

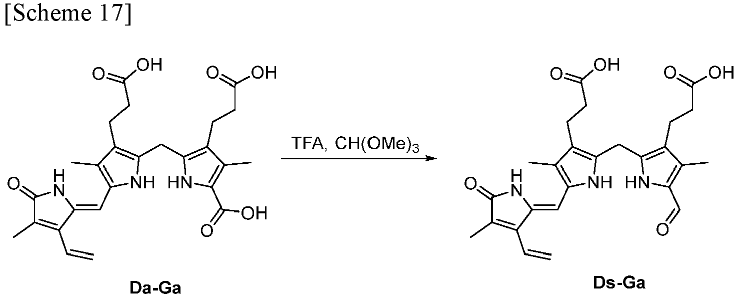

The present invention provides a method for synthesizing bilirubin, which further includes coupling a compound represented by Formula 7 with the compound represented by Formula 4 to prepare the compound represented by Formula 6.

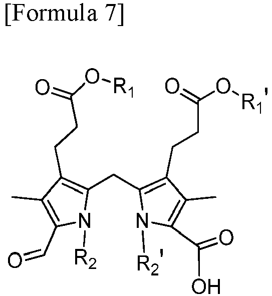

In Formula 7, R₁, R₁', R₂ and R₂' are the same as R₁, R₁', R₂ and R₂' in Formula 6, respectively.

The coupling reaction is carried out under the same solvent and base as the coupling reaction described above.

The coupling reaction is carried out in the same reaction temperature and time range as the coupling reaction described above.

The coupling reaction between the compound represented by Formula 7 with the compound represented by Formula 4 is, for example, shown in Schemes 18 and 19 below.

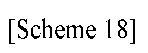

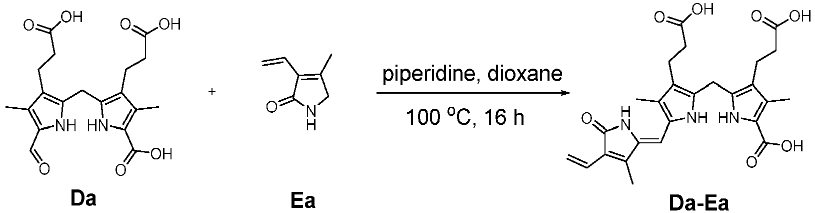

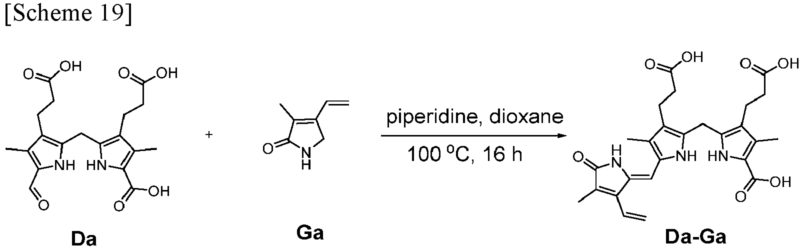

The present invention provides a method for synthesizing bilirubin, which includes a step of reacting a compound represented by Formula 1 with a compound represented by Formula 10 to prepare a compound represented by Formula 6.

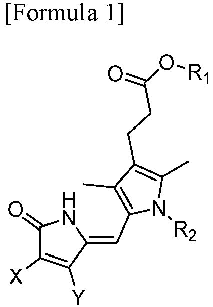

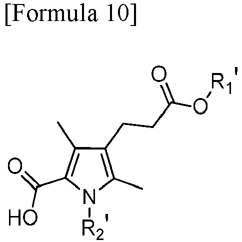

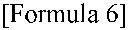

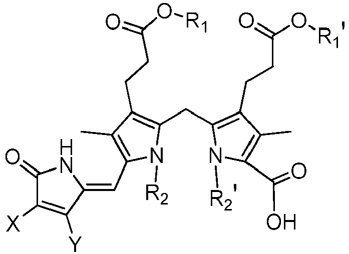

Formula 6 is the same as defined above.

In Formulas 1 and 10, R₁, R₁', R₂, R₂', X and Y are the same as R₁, R₁', R₂, R₂', X and Y in Formula 6, respectively.

The reaction between the compound represented by Formula 1 and the compound represented by Formula 10 below may be carried out under bromine condition.

The reaction between the compound represented by Formula 1 and the compound represented by Formula 10 may be carried out under the same condition as the solvent, reaction temperature, and reaction time in the dimerization reaction described above.

The method for synthesizing bilirubin of the present invention may include a step of PEGylating the compound represented by Formula 1 with polyethylene glycol (PEG) and then reacting the resulting product with the compound represented by Formula 10.

The reaction between the compound represented by Formula 1 and the compound represented by Formula 10 is, for example, shown in Schemes 20 and 21 below.

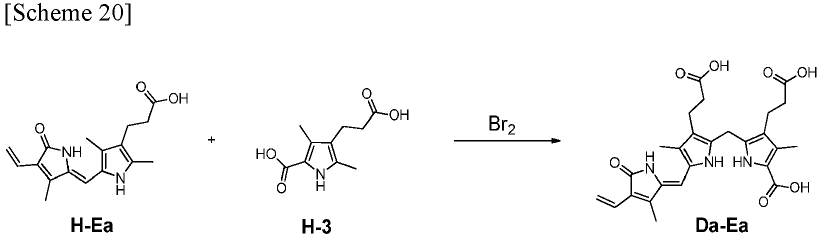

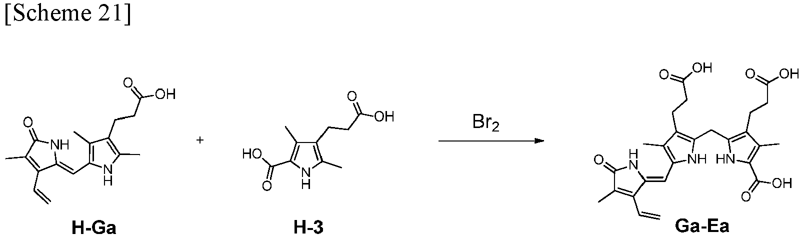

The present invention relates to a method for synthesizing bilirubin, which includes a step of coupling a compound represented by Formula 8 with a compound represented by Formula 4 to prepare a compound represented by Formula 3 below.

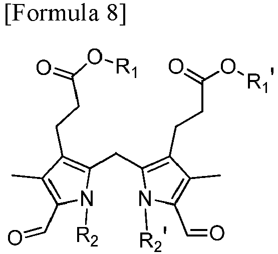

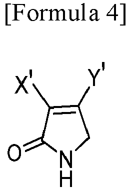

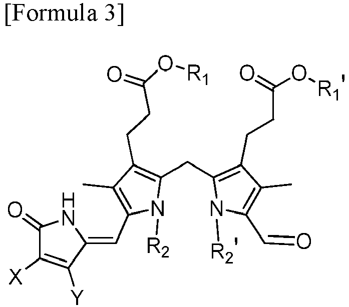

Formula 3 is the same as defined above.

In Formulas 8 and 4, R₁, R₁', R₂, R₂', X' and Y' are the same as R₁, R₁', R₂, R₂', X and Y in Formula 3, respectively.

The coupling reaction is carried out under the same solvent and base as the coupling reaction described above.

The coupling reaction is carried out in the same reaction temperature and time range as the coupling reaction.

The method for synthesizing bilirubin of the present invention may include a step of PEGylating the compound represented by Formula 8 with polyethylene glycol (PEG) and then coupling the resulting product with the compound represented by Formula 4.

The PEGylation reaction is carried out under the same solvent and base as the PEGylation reaction described above.

The PEGylation reaction is carried out in the same reaction temperature and time range as the PEGylation reaction described above.

The present invention relates to a method for synthesizing bilirubin, which includes a step of substituting a carboxyl group bonded to a pyrrole group of the compound represented by Formula 9 with an aldehyde group to prepare a compound represented by Formula 8.

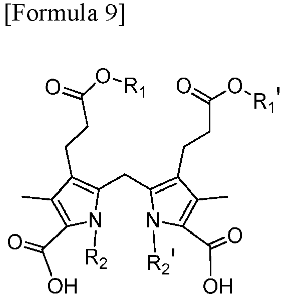

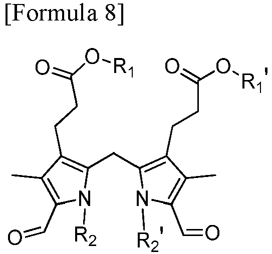

Formula 8 is the same as defined above.

In Formula 9, R₁, R₁', R₂ and R₂' are the same as R₁, R₁', R₂ and R₂' in Formula 8, respectively.

A carboxyl group is reduced by a known method, or a carboxyl group is removed and then an aldehyde group is added to substitute the carboxyl group with the aldehyde group. A carboxyl group may be substituted with an aldehyde group, for example, under trimethoxymethane and TFA.

A reaction, in which the carboxyl group bonded to the pyrrole group of the compound represented by Formula 9 is substituted with an aldehyde group, is shown in Scheme 22 below.

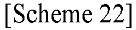

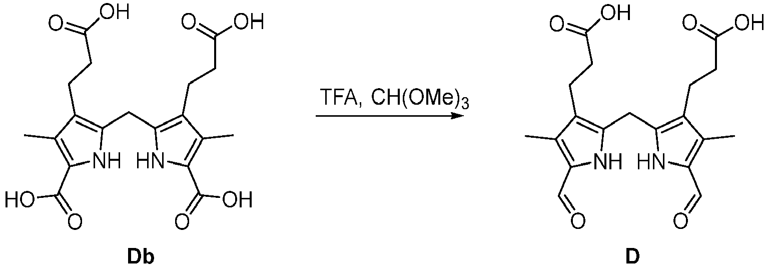

The present invention relates to a method for synthesizing bilirubin, which includes a step of substituting a carboxyl group bonded to a pyrrole group of a compound represented by Formula 7 below with an aldehyde group to prepare a compound represented by Formula 8.

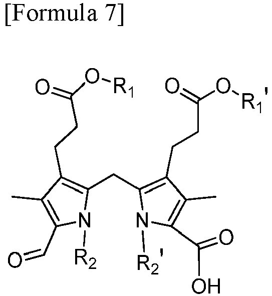

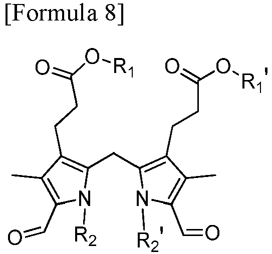

Formula 8 is the same as defined above.

In Formula 7, R₁, R₁', R₂ and R₂' are the same as R₁, R₁', R₂ and R₂' in Formula 8, respectively.

A carboxyl group is reduced by a known method, or a carboxyl group is removed and then an aldehyde group is added to substitute the carboxyl group with the aldehyde group. A carboxyl group may be substituted with an aldehyde group, for example, under trimethoxymethane and TFA.

A reaction, in which the carboxyl group bonded to the pyrrole group of the compound represented by Formula 7 is substituted with an aldehyde group, is shown in Scheme 23 below.

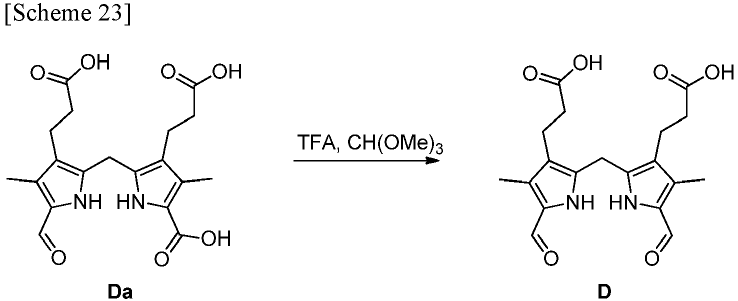

The present invention relates to a method for synthesizing bilirubin, which includes a step of substituting one carboxyl group bonded to a pyrrole group of a compound represented by Formula 9 below with an aldehyde group to prepare a compound represented by Formula 7.

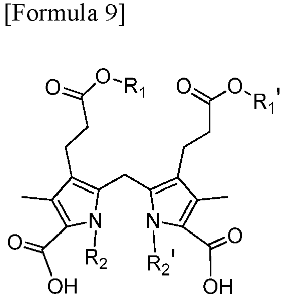

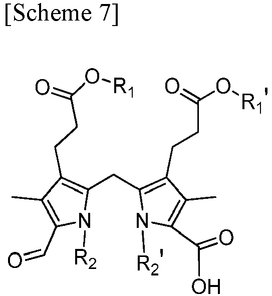

Formula 7 is the same as defined above.

In Formula 9, R₁, R₁', R₂ and R₂' are the same as R₁, R₁', R₂ and R₂' in Formula 2, respectively.

A carboxyl group is reduced by a known method, or a carboxyl group is removed and then an aldehyde group is added to substitute the carboxyl group with the aldehyde group. A carboxyl group may be substituted with an aldehyde group, for example under trimethoxymethane and TFA.

The present invention relates to a method for synthesizing bilirubin, which includes a step of reacting a compound represented by Formula 10 with a compound represented by Formula 5 to prepare a compound represented by Formula 7.

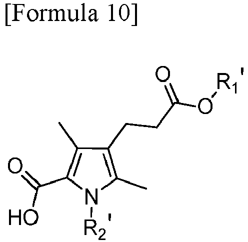

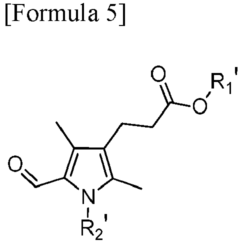

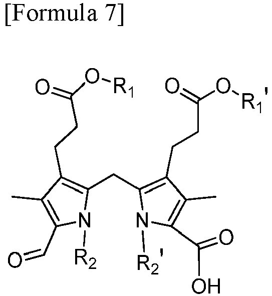

Formula 7 is the same as defined above.

In Formulas 10 and 5, R₁, R₁', R₂ and R₂' are the same as R₁, R₁', R₂ and R₂' of Formula 7, respectively.

The reaction between the compound represented by Formula 10 and the compound represented by Formula 5 may be carried out under bromine or chloranil condition.

The reaction between the compound represented by Formula 10 and the compound represented by Formula 5 may be performed under the same condition as the solvent, reaction temperature, and reaction time in the dimerization reaction.

The method for synthesizing bilirubin of the present invention may include a step of PEGylating the compound represented by Formula 10 with polyethylene glycol (PEG) and then reacting the resulting product with the compound represented by Formula 5.

The method for synthesizing bilirubin of the present invention may include a step of PEGylating the compound represented by Formula 5 with polyethylene glycol (PEG) and then reacting the resulting product with the compound represented by Formula 10.

The PEGylation reaction is carried out under the same solvent and base as the PEGylation reaction described above.

The PEGylation reaction is carried out in the same reaction temperature and time range as the PEGylation reaction described above.

The reaction between the compound represented by Formula 10 and the compound represented by Formula 5 is, for example, shown in Scheme 24 below.

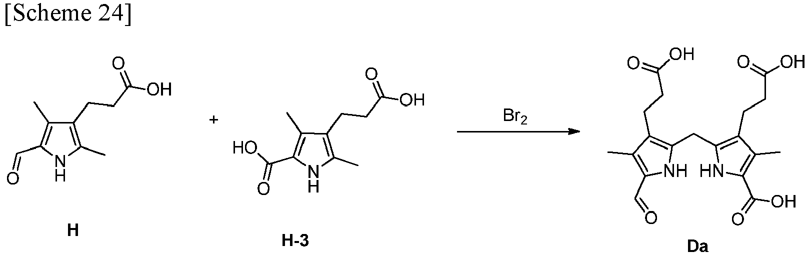

The present invention relates to a method for synthesizing bilirubin, which includes a step of dimerizing a compound represented by Formula 5 to prepare a compound represented by Formula 8.

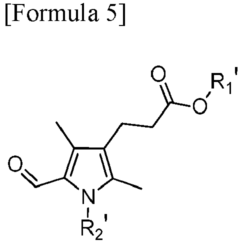

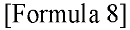

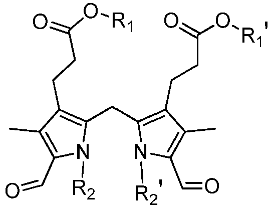

In Formulas 5 and 8, R₁, R₁', R₂ and R₂' are the same as R₁, R₁', R₂ and R₂' of Formula 3, respectively.

The dimerization reaction of the compound represented by Formula 5 may be performed under bromine or chloranil conditions.

The dimerization reaction is carried out in the same solvent as the dimerization reaction described above.

The dimerization reaction is carried out at the same reaction temperature and time range as the dimerization reaction described above.

The present invention relates to a method for synthesizing bilirubin, which includes a step of dimerizing a compound represented by Formula 10 to prepare a compound represented by Formula 9.

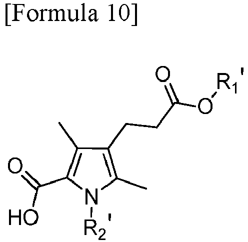

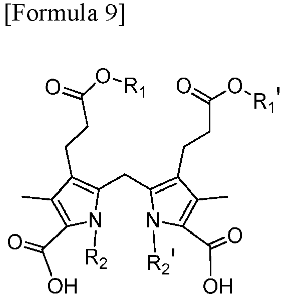

In Formulas 10 and 9, R₁, R₁', R₂ and R₂' are the same as R₁, R₁', R₂ and R₂' in Formula 3, respectively.

The dimerization reaction of the compound represented by Formula 10 may be performed under bromine or chloranil conditions.

The dimerization reaction is carried out in the same solvent as the dimerization reaction described above.

The dimerization reaction is carried out at the same reaction temperature and time range as the dimerization reaction described above.

The dimerization reaction of the compound represented by Formula 10 is, for example, shown in Scheme 25 below.

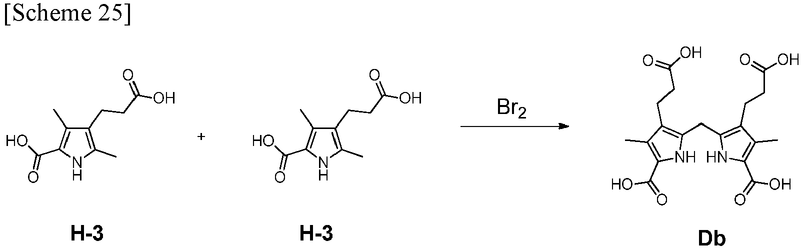

The present invention relates to a method for synthesizing bilirubin, which includes a step of coupling a compound represented by Formula 5 with a compound represented by Formula 4 to prepare a compound represented by Formula 1.

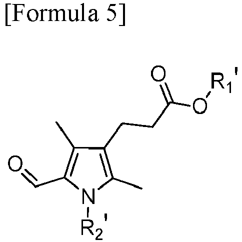

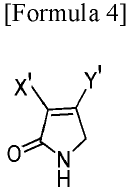

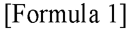

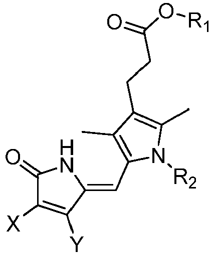

Formula 1 is the same as defined above.

In Formulas 5 and 4, R₁', R₂', X' and Y' are the same as R₁, R₂, X and Y in Formula 1, respectively.

The coupling reaction is carried out under the same solvent and base as the coupling reaction described above.

The coupling reaction is carried out in the same reaction temperature and time range as the above-described coupling reaction.

The coupling reaction between the compound represented by Formula 5 and the compound represented by Formula 4 below is shown in Schemes 26 and 27 below.

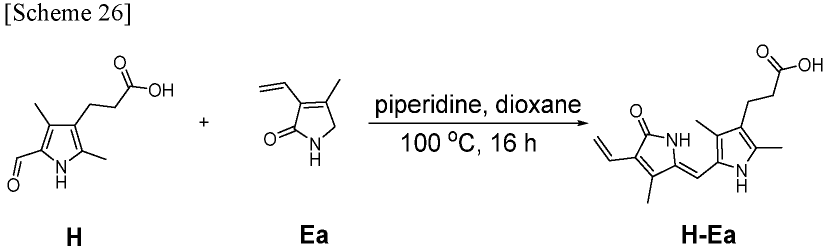

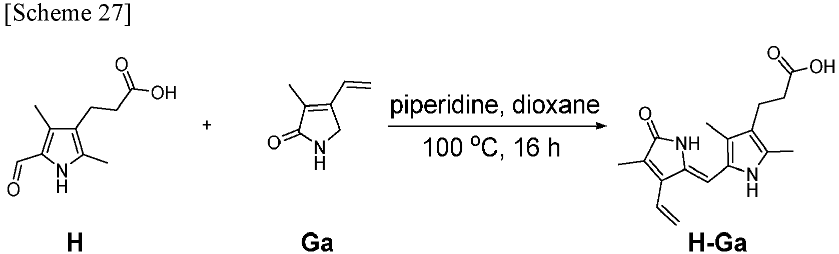

Hereinafter, the present invention will be described in more detail through examples.

### <EXAMPLE>

### 1. Preparation of compound represented by Formula 1

A compound corresponding to the compound represented by Formula 1 herein was prepared by coupling the compound represented by Formula 4 and the compound represented by Formula 5 herein as follows.

### 1.1. Preparation of compound represented by Formula 4

Compounds Ea, Ga, Ea-2a and Ed corresponding to the compound represented by Formula 4 herein were prepared as follows (Examples 1 to 4).

### Example 1: Preparation of Compound Ea

### (1-1) Preparation of Compound Ea-1

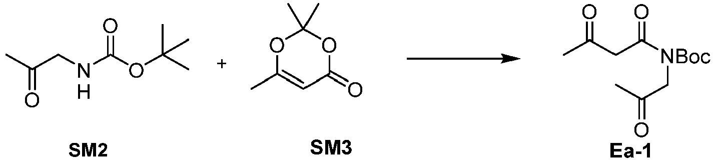

To a mixture of compound SM2 (40.0 g, 231 mmol, 1.0 equiv.) in xylene (241 mL), compound SM3 (32.8 g, 231 mmol, 1.0 equiv.) was added. The mixture was stirred at 150 °C for 10 minutes under nitrogen condition. The mixture was concentrated under reduced pressure condition. The residue was purified by silica gel chromatography to yield Compound Ea-1 in the form of brown oil (51.0 g, 198 mmol, yield: 85%).

¹H NMR (400 MHz, CDCl₃) δ 4.48 (s, 2H), 4.00 (s, 2H), 2.21 (s, 3H), 2.13 (s, 3H), 1.39 (s, 9H).

### (1-2) Preparation of Compound Ea-2

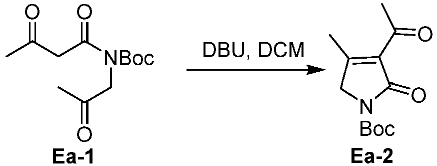

A mixture of DBU (34.8 mL, 233 mmol, 0.5 equiv.) in DCM (348 mL) was added dropwise to a mixture of Compound Ea-1 (120 g, 466 mmol, 1.0 equiv.) prepared above in DCM (600 mL) at 0 °C and stirred for 40 minutes. The reaction was terminated with KH₂PO₄ aqueous solution (480 mL). Then, the organic layer was extracted with DCM (600 mL x 2) and washed with water (480 mL) and brine (1.2 L). The mixed organic layer was dried over anhydrous Na₂SO₄, filtered and concentrated under reduced pressure condition. The residue was dissolved in MTBE (60 mL) and Petroleum ether (720 mL) was added thereto at 0 °C. The resulting precipitate was filtered under reduced pressure condition to yield Compound Ea-2 as a brown solid (90 g, 372 mmol, yield: 80%).

¹H NMR (400 MHz, CDCl₃) δ 4.27 (s, 2H), 2.56 (s, 3H), 2.39 (s, 3H), 1.57 (s, 9H).

### (1-3) Preparation of Compound Ea-3

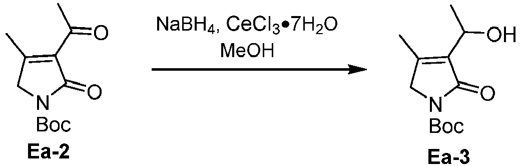

CeCl₃·7H₂O (126g, 338mmol, 2.0 equiv.) was dissolved in methanol (600 mL) and stirred for 5 minutes. The Compound Ea-2 (53.9 g, 16 9mmol, 1.0 equiv.) prepared above was added to the mixture and stirred for 5 minutes. Thereafter, the mixture was cooled to 0 °C and NaBH₄ (12.8 g, 338 mmol, 2.0 equiv.) was added slowly thereto for about 1 hour. The mixture was stirred at 0 °C for 3 hours under nitrogen (N₂) condition. 1 M HCl aqueous solution (300 mL) was added to the mixture, followed by extraction with EtOAc (500 mL × 5). The mixed organic layer was washed with brine (300 mL), dried over anhydrous Na₂SO₄, filtered and concentrated under reduced pressure condition to obtain a residue. The residue was purified by flash silica gel chromatography to yield Compound Ea-3 in the form of brown oil (23.0 g, 95.3 mmol, yield: 56%).

¹H NMR (400 MHz, CDCl₃) δ 4.72 - 4.64 (m, 1H), 4.13 (s, 2H), 3.49 (d, *J* = 9.6 Hz, 1H), 2.05 (s, 3H), 1.56 (s, 9H), 1.48 (d, *J* = 6.8 Hz, 3H).

### (1-4) Preparation of Compound Ea-4

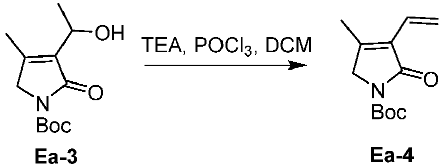

To a mixture of Compound Ea-3 (23.0 g, 95.3 mmol, 1.0 equiv.) prepared above in DCM (700 mL), a mixture of TEA (116 g, 1.14 mol, 12.0 equiv.) andPOCl₃ (58.4 g, 381 mmol, 4.0 equiv.) in DCM (180 mL) was added dropwise at 0 °C for 1 hour. The mixture was stirred at 20 °C for 3 hours under nitrogen (N₂) condition, and monitored by TLC. The mixture was concentrated under reduced pressure condition, diluted with water (300 mL) and extracted with DCM (500 mL × 2). The mixed organic layer was washed with brine (200 mL), dried over anhydrous Na₂SO₄, filtered and concentrated under reduced pressure condition to obtain a residue. The residue was purified by flash silica gel chromatography to yield Compound Ea-4 in the form of yellow solid (8.0 g, 24.2 mmol, yield: 38%).

¹H NMR (400 MHz, CDCl₃) δ 6.45 - 6.38 (m, 1H), 6.31 - 6.26 (m, 1H), 5.46 - 5.42 (m, 1H), 4.15 (s, 2H), 2.11 (s, 3H), 1.56 (s, 9H).

### (1-5) Preparation of Compound Ea

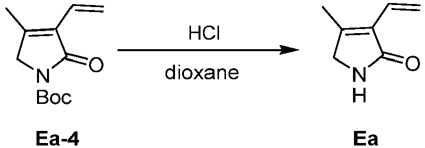

HCl/1,4-dioxane (4M, 18 mL) was added to a mixture of the Compound Ea-4 (4.0 g, 17.9 mmol) prepared above in EtOAc (18 mL) at 0 °C, and the mixture was stirred at 20 °C for 1 hour. The reaction was terminated by adding aqueous NaHCO₃ solution (80 mL) to the mixture. The mixture was extracted with EtOAc (80 mL x 2). The mixed organic layer was washed with brine (100 mL), dried over anhydrous Na₂SO₄, filtered and concentrated to form Compound Ea corresponding to the compound represented by Formula 4 herein in the form of yellow solid (4.6 g, yield: >99 %).

¹H NMR (400 MHz, CDCl₃) δ 6.87 (brs, 1H), 6.48 (dd, *J* = 17.6, 11.6 Hz, 1H), 6.24 (dd, *J* = 18.0, 2.0 Hz, 1H), 5.41 (dd, *J* = 11.6, 2.0 Hz, 1H), 3.86 (s, 2H), 2.09 (s, 3H).

### Example 2: Preparation of Compound Ga

### (2-1) Preparation of Compound Ga-2

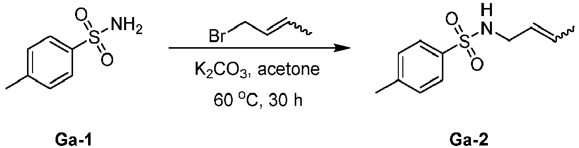

To a mixture of Compound Ga-1 (55.8 g, 326 mmol, 1.1 equiv.) in acetone (400 mL), K₂CO₃ (45.0 g, 326 mmol, 1.1 equiv.) and 1-bromobut-2-ene (40.0 g, 296 mmol, 1.0 equiv.) were added and stirred at 60 °C for 30 hours under nitrogen condition. The mixture was filtered and concentrated under reduced pressure condition. The residue was purified through flash silica gel chromatography to yield Compound Ga-2 as a yellow solid (42.4 g, yield: 64%).

¹H NMR (400 MHz, CDCl₃) δ 7.75 (d, *J* = 8.4 Hz, 2H), 7.30 (d, *J* = 8.0 Hz, 2H), 5.59 - 5.51 (m, 1H), 5.36 - 5.28 (m, 1H), 4.95 - 4.78 (m, 1H), 3.62 - 3.48 (m, 2H), 2.43 (s, 3H), 1.60 - 1.53 (m, 3H).

### (2-2) Preparation of Compound Ga-3

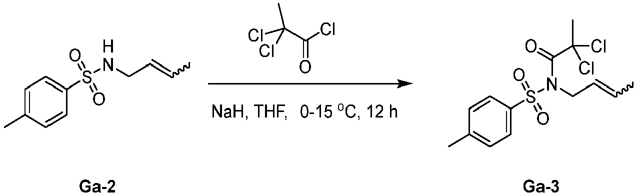

To a mixture of Compound Ga-2 (5.00 g, 22.2 mmol, 1.0 equiv.) in THF (50 mL), NaH (1.33 g, 33.3 mmol, 60% purity in mineral oil, 1.5 equiv.) was added slowly at 0 °C. To the mixture, a mixture of dichloropropanoyl chloride (9.79 g, 60.7 mmol, 2.7 equiv.) in DCM (15 mL) was added at 0 °C, and the mixture was stirred at 15 °C for 12 hours. At 25 °C, water (100 mL) was added to the mixture, followed by extraction with DCM (200 mL x 2). The mixed organic layer was dried over anhydrous Na₂SO₄, filtered and concentrated under reduced pressure condition. The residue was purified by flash silica gel chromatography to yield Compound Ga-3 as a yellow solid (4.47 g, yield: 21%).

¹H NMR (400 MHz, CDCl₃) δ 7.80 (d, *J* = 8.4 Hz, 2H), 7.24 (d, *J* = 8.0 Hz, 2H), 5.90 - 5.81 (m, 1H), 5.60 - 5.50 (m, 1H), 4.99 - 4.86 (m, 2H), 2.37 (s, 3H), 2.13 (s, 3H), 1.75 - 1.69 (m, 3H).

### (2-3) Preparation of Compound Ga-4

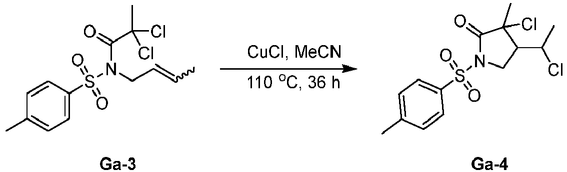

To a mixture of Compound Ga-3 (9.29 g, 26.5 mmol, 1.0 equiv.) in ACN (40 mL), CuCl (1.05 g, 10.6 mmol, 0.4 equiv.) was added and stirred at 110 °C for 36 hours under nitrogen condition. The mixture was concentrated under reduced pressure condition. The residue was purified through flash silica gel chromatography to yield Compound Ga-4 in the form of yellow oil (8.50 g, yield: 91%).
C₁₄H₁₇Cl₂NO₃S m/z [M+H]⁺ = 350.0

### (2-4) Preparation of Compound Ga-5

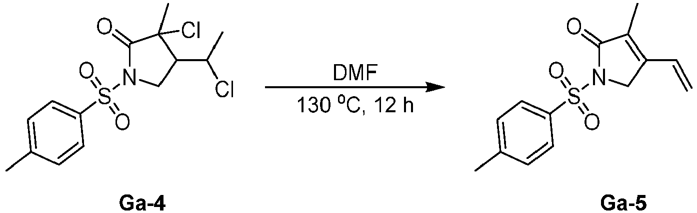

A mixture of Compound Ga-4 (8.50 g, 24.3 mmol, 1.0 equiv.) in DMF (90 mL) was stirred at 130 °C for 12 hours under nitrogen condition. After adding EtOAc (250 mL) to the mixture, it was washed with water (50 mL x 4). The mixed organic layer was washed with brine (50 mL x 2), dried over anhydrous Na₂SO₄, filtered and concentrated under reduced pressure condition. The residue was purified through flash silica gel chromatography to yield Compound Ga-5 as a yellow solid (4.70 g, 17.0 mmol, yield: 70%).

¹H NMR (400 MHz, CDCl₃) δ 7.90 (d, *J* = 8.4 Hz, 2H), 7.26 (d, *J* = 8.0 Hz, 2H), 6.62 (dd, *J* = 17.6, 11.2 Hz, 1H), 5.51 (d, *J* = 18.0 Hz, 1H), 5.43 (d, *J* = 10.8 Hz, 1H), 4.41 (d, *J* = 1.2 Hz, 2H), 2.36 (s, 3H), 1.76 (s, 3H).

### (2-5) Preparation of Compound Ga

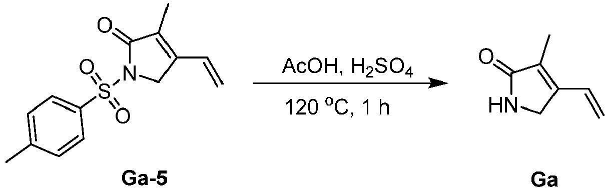

A mixture of HOAc (9.46 g, 158 mmol, 8.7 equiv.) of Compound Ga-5 (5.00 g, 18.0 mmol, 1.0 equiv.) and conc. H₂SO₄ (16.6 g, 162 mmol, 9.0 equiv.) was stirred at 100 °C for 1 hour under nitrogen condition. 10% Na₂CO₃ aqueous solution (300 mL) was added to the mixture, followed by extraction with DCM (100 mL x 5). The mixed organic layer was dried over anhydrous Na₂SO₄, filtered and concentrated under reduced pressure condition to yield Compound Ga corresponding to the compound represented by Formula 4 herein as a yellow solid (1.18 g, 9.58 mmol, yield: 53%).

¹H NMR (400 MHz, CDCl₃) δ 6.79 (brs, 1H), 6.73 (dd, *J* = 17.6, 11.2 Hz, 1H), 5.45 (d, *J* = 17.6 Hz, 1H), 5.37 (d, *J* = 10.8 Hz, 1H), 4.06 (s, 2H), 1.92 (s, 3H).
C₇H₉NO m/z [M+H]⁺ = 124

### Example 3: Preparation of Compound Ea-2a

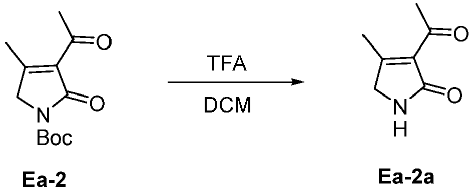

After adding TFA (0.32 mL, 4.18 mmol, 5.0 equiv.) to a mixture of Compound Ea-2 (200 mg, 0.83 mmol, 1.0 equiv.) prepared above in DCM (16 mL), the mixture was stirred at 25 °C for 1 hour. The pH was adjusted to 7 by adding aqueous NaHCO₃ solution (50 mL) thereto. After extraction with DCM (50 mL × 3), the mixed organic layer was dried over anhydrous Na₂SO₄, filtered and concentrated under reduced pressure condition. After adding DCM (3 mL) to the concentrated filtrate and stirring at 25 °C for 5 minutes, hexane (20 mL) was added dropwise, and then filtered under reduced pressure to yield Compound Ea-2a corresponding to the compound represented by Formula 4 herein as a yellow solid (70 mg, 0.50 mmol, yield: 60%).

¹H NMR (400 MHz, CDCl₃) δ 7.15 (brs, 1H), 3.97 (s. 2H), 2.55 (s, 3H), 2.36 (s, 3H).
C₇H₉NO₂ m/z [M+H]⁺ = 140

### Example 4: Preparation of Compound Ed

### (4-1) Preparation of Compound Ed-2

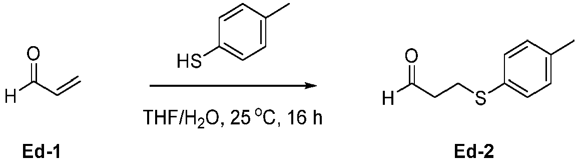

Ed-1 (20.1 g, 358 mmol, 1.0 equiv.) was added dropwise to a mixture of 4-methylbenzenethiol (36.9 g, 297 mmol, 0.83 equiv.) in THF (300 mL) and water (150 mL) at 0 °C, and then stirred at 25 °C for 16 hours under nitrogen condition. NaHCO₃ aqueous solution (200 mL) was added to the reaction mixture, followed by extraction with EtOAc (200 mL × 2). The mixed organic layer was washed with brine (50 mL × 2), dried over anhydrous Na₂SO₄, filtered and concentrated under reduced pressure condition to yield Compound Ed-2 in the form of yellow oil (59.4 g, 330 mmol, yield: 92 %).
¹H NMR (400 MHz, CDCl₃) δ 9.74 (s, 1H), 7.27 (d, *J* = 8.0 Hz, 2H), 7.11 (d, *J* = 7.6 Hz, 2H), 3.13 (t, *J* = 7.2 Hz, 2H), 2.75 - 2.71 (m, 2H), 2.32 (s, 3H),

### (4-2) Preparation of Compound Ed-3

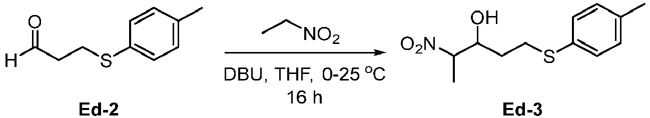

To a mixture of Compound Ed-2 (58 g, 322 mmol, 1.0 equiv.) and DBU (4.90 g, 32.2 mmol, 0.1 equiv.) in THF (400 mL), a mixture of 1-nitroethane (24.0 g, 322 mmol, 1.0 equiv.) in THF (50 mL) was added at 0 °C and stirred at 25 °C for 16 hours. The mixture was diluted with water (200 mL) then extracted with EtOAc (400 mL x 2). The mixed organic layer was washed with brine (200 mL), dried over anhydrous Na₂SO₄, filtered and concentrated under reduced pressure condition to obtain a residue. The residue was purified by flash silica gel chromatography to yield Compound Ed-3 in the form of yellow oil (51.7 g, 202 mmol, yield: 63%).

¹H NMR (400 MHz, CDCl₃) δ 7.28 (d, *J* = 7.6 Hz, 2H), 7.13 (d, *J* = 8.0 Hz, 2H), 4.54 - 4.46 (m, 1H), 4.15 - 4.12 (m, 1H), 3.15 - 3.08 (m, 1H), 3.03 - 2.96 (m, 1H), 2.33 (s, 3H), 1.64-1.80 (m, 2H), 1.53 (t, *J* = 8.0 Hz, 3H).

### (4-3) Preparation of Compound Ed-3

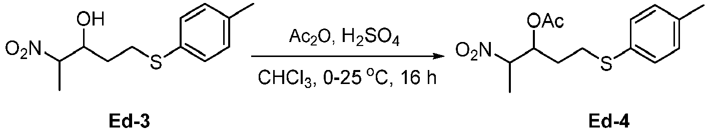

Acetic anhydride (31.0 g, 304 mmol, 1.5 equiv.) was added dropwise to a mixture of Compound Ed-3 (51.7 g, 202 mmol, 1.0 equiv.) and H₂SO₄ (199 mg, 2.02 mmol, 0.01 equiv.) in CHC₃ (500 mL) at 0 °C and stirred at 25 °C for 16 hours. The reaction was terminated by adding NaHCO₃ aqueous solution (100 mL) to the reaction mixture, followed by extraction with DCM (50 mL × 4). The mixed organic layer was washed with brine (50 mL × 2), dried over anhydrous Na₂SO₄, filtered and concentrated under reduced pressure condition to yield Compound Ed-4 in the form of brown oil (65.5 g, yield: >99%).

¹H NMR (400 MHz, CDCl₃) δ 7.27 (d, *J* = 8.4 Hz, 2H), 7.12 (d, *J* = 8.0 Hz, 2H), 5.45 - 5.40 (m, 1H), 4.75 - 4.66 (m, 1H), 2.98 - 2.78 (m, 2H), 2.33 (s, 3H), 2.07 (d, *J* = 8.8 Hz, 3H), 1.99 - 1.80 (m, 2H), 1.49 (dd, *J=* 6.8, 2.0 Hz, 3H).

### (4-4) Preparation of Compound Ed-5

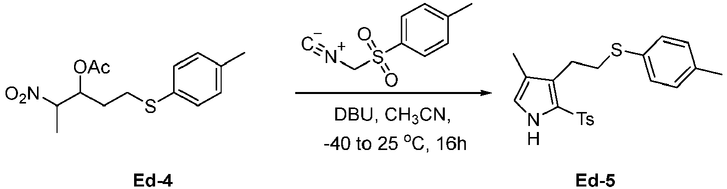

To a mixture of Compound Ed-4 (3.61 g, 18.5 mmol, 1.0 equiv.) and DBU (5.63 g, 37.0 mmol, 2.0 equiv.) in ACN (50 mL), a mixture of TosMIC (5.00 g, 16.8 mmol, 0.9 equiv.) in ACN (10 mL) was added dropwise at -40 °C under nitrogen condition. The mixture was stirred at 25 °C for 16 hours under nitrogen condition. The mixture was diluted with water (100 mL) and then extracted with EtOAc (100 mL × 2). The mixed organic layer was washed with brine (100 mL), dried over anhydrous Na₂SO₄, filtered and concentrated under reduced pressure condition to obtain a residue. The residue was purified by flash silica gel chromatography to yield Compound Ed-5 in the form of red oil (3.47 g, 9.00 mmol, yield: 53%).

¹H NMR (400 MHz, CDCl₃) δ 8.99 (s, 1H), 7.65 (d, *J=* 8.0 Hz, 2H), 7.31 (d, *J* = 8.0 Hz, 2H), 7.21 (d, *J* = 8.0 Hz, 2H), 7.14 (d, *J* = 8.0 Hz, 2H), 6.69 (d, *J* = 2.0 Hz, 1H), 2.87 - 2.81 (m, 4H), 2.37 (s, 3H), 2.35 (s, 3H), 1.93 (s, 3H).

### (4-5) Preparation of Compound Ed-6

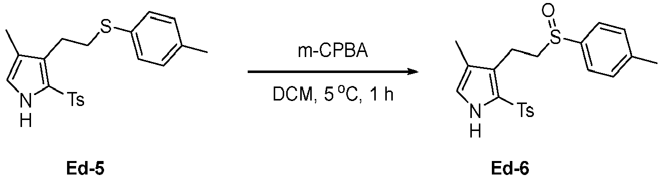

To a mixture of Compound Ed-5 (10.0 g, 25.9 mmol, 1.0 equiv.) in DCM (40 mL), mCPBA (5.27 g, 25.9 mmol, 85% purity, 1.0 equiv.) was added dropwise at 5 °C and the mixture was stirred at 5°C for 1 hour under nitrogen condition. After the reaction was terminated by adding Na₂SO₃ aqueous solution (100 mL) to the mixture, the mixture was extracted with DCM (50 mL × 2). The mixed organic layer was washed with brine (100 mL), dried over anhydrous Na₂SO₄, filtered and concentrated under reduced pressure condition to obtain a residue. The residue was purified by flash silica gel chromatography to yield Compound Ed-6 in the form of white solid (5.0 g, 12.5 mmol, yield: 48%).

¹H NMR (400 MHz, CDCl₃) δ 9.20 (s, 1H), 7.57 - 7.52 (m, 4H), 7.36 (d, *J* = 8.4 Hz, 2H), 7.21 (d, *J* = 8.0 Hz, 2H), 6.69 (d, *J* = 2.8 Hz, 1H), 2.97 - 2.93 (m, 2H), 2.89 - 2.69 (m, 2H), 2.45 (s, 3H), 2.39 (s, 3H), 1.94 (s, 3H).

### (4-6) Preparation of Compound Ed-7

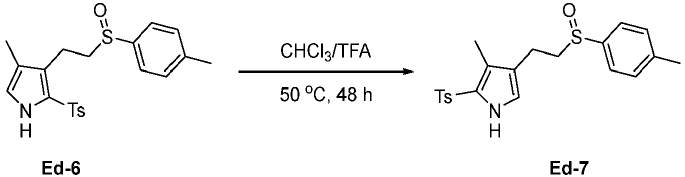

A mixture of Compound Ed-6 (5.0 g, 12.5 mmol, 1.0 equiv.) and TFA (5 mL) in CHCl₃ (45 mL) was stirred at 50 °C for 48 hours under nitrogen condition. The reaction was terminated by adding water (100 mL) to the mixture and then extracted with DCM (100 mL × 2). The mixed organic layer was washed with brine (50 × 2 mL), dried over anhydrous Na₂SO₄, filtered and concentrated under reduced pressure condition to obtain a residue. The residue was purified by flash silica gel chromatography to yield Compound Ed-7 in the form of white solid (1.90 g, 4.68 mmol, yield: 37.6%).

¹H NMR (400 MHz, CDCl₃) δ 9.02 (s, 1H), 7.75 (d, *J* = 8.4 Hz, 2H), 7.49 (d, *J* = 8.4 Hz, 2H), 7.33 - 7.27 (m, 4H), 6.74 (d, *J* = 2.8 Hz, 1H), 2.91 - 2.64 (m, 4H), 2.42 (s, 3H), 2.40 (s, 3H), 2.10 (s, 3H).

### (4-7) Preparation of Compound Ed-8

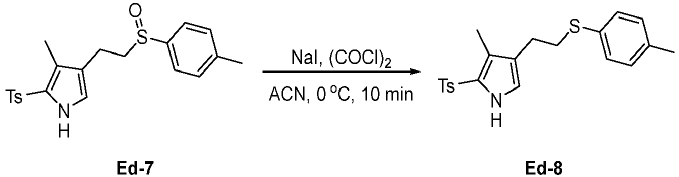

To a mixture of Compound Ed-7 (3.0 g, 7.47 mmol, 1.0 equiv.) in ACN (48 mL), NaI (2.82 g, 18.67 mmol, 2.5 equiv.) was added at 0 °C and stirred for 10 minutes. (COCl)₂ (0.77 ml, 9.38 mmol, 1.2 equiv.) was added dropwise to the mixture at 0 °C and stirred for 10 minutes under the same condition. The reaction was terminated with water, followed by extraction with EtOAc (50 mL × 2). The mixed organic layer was washed with brine (100 mL), dried over anhydrous Na₂SO₄, filtered and concentrated under reduced pressure condition. The residue was purified by silica gel chromatography to yield Compound Ed-8 in the form of brown solid (2.21 g, 5.71 mmol, yield: 76%).

¹H NMR (400 MHz, CDCl₃) δ 8.83 (s, 1H), 7.76 (d, *J* = 8.0 Hz, 2H), 7.29 (d, *J* = 8.0 Hz, 2H), 7.23 (d, *J* = 8.4 Hz, 2H), 7.09 (d, *J* = 8.0 Hz, 2H), 6.75 (d, *J* = 2.8 Hz, 1H), 3.00 - 2.96 (m, 2H), 2.65 (t, *J* = 8.0 Hz, 2H), 2.41 (s, 3H), 2.32 (s, 3H), 2.12 (s, 3H).

### (4-8) Preparation of Compound Ed-9

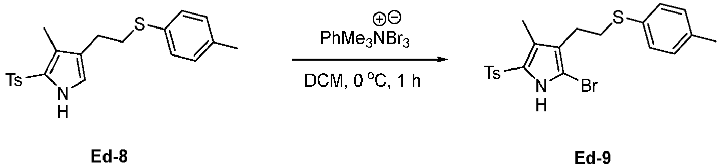

To a mixture of Compound Ed-8 (2.2 g, 5.71 mmol, 1.0 equiv.) in DCM (62 mL), PhMe₃NBr₃ (2.36 g, 5.71 mmol, 1.0 equiv.) was added at 0 °C and stirred for 1 hour. NaHSO₃ aqueous solution (30 mL) was added to the mixture, followed by extraction with DCM (50 mL × 2). The mixed organic layer was washed with brine (100 mL), dried over anhydrous Na₂SO₄, filtered and concentrated under reduced pressure condition. The residue was purified by silica gel chromatography to yield Compound Ed-9 in the form of brown solid (2.65, 5.23 mmol, yield: 99%).

¹H NMR (400 MHz, CDCl₃) δ 8.93 (s, 1H), 7.76 (d, *J* = 8.0 Hz, 2H), 7.30 (d, *J* = 8.4 Hz, 2H), 7.25 (d, *J* = 8.0 Hz, 2H), 7.09 (d, *J* = 8.0 Hz, 2H), 2.92 (t, *J* = 7.6 Hz, 2H), 2.62 (t, *J* = 7.6 Hz, 2H), 2.42 (s, 3H), 2.32 (s, 3H), 2.13 (s, 3H).

### (4-9) Preparation of Compound Ed-10

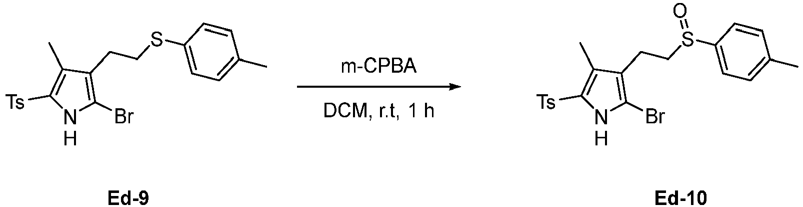

To a mixture of Compound Ed-9 (2.59 g, 5.57 mmol, 1.0 equiv.) in DCM (50 mL), mCPBA (1.05 g, 6.13 mmol, 1.1 equiv.) was added at 0 °C and stirred at 25 °C for 1 hour. NaHSO₃ aqueous solution (30 mL) was added to the mixture, followed by extraction with DCM (60 mL × 2). The mixed organic layer was washed with brine (100 mL), dried over anhydrous Na₂SO₄, filtered and concentrated under reduced pressure condition. The residue was solidified using DCM/Hexanes to yield Compound Ed-10 in the form of white solid (2.9 g, yield: >99%).

¹H NMR (500 MHz, CDCl₃) δ 9.00 (s, 1H), 7.68 (d, *J* = 8.0 Hz, 2H), 7.43 (d, *J* = 7.9 Hz, 2H), 7.24 (dd, *J* = 12.2, 8.0 Hz, 4H), 2.86 - 2.78 (m, 1H), 2.78 - 2.67 (m, 2H), 2.58 - 2.48 (m, 1H), 2.34 (s, 3H), 2.33 (s, 3H) 2.04 (s, 3H).

### (4-10) Preparation of Compound Ed-11

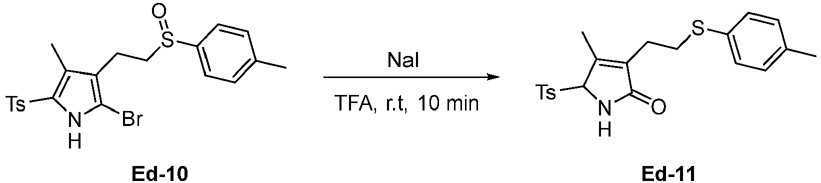

A mixture of Compound Ed-10 (1.0 g, 2.08 mmol, 1.0 equiv.) and TFA (1.5 mL) was stirred at 25 °C for 30 min under nitrogen condition. After adding NaI (1.56 g, 10.4 mmol, 5.0 equiv.), the mixture was stirred at 25 °C for 10 minutes, neutralized by adding K₂CO₃ aqueous solution at 0 °C, followed by extraction with DCM (150 mL × 2). The mixed organic layer was dried over anhydrous Na₂SO₄, filtered and concentrated under reduced pressure condition. The residue was purified by silica gel chromatography to yield dark green Compound Ed-11 (550 mg, 1.37 mmol, yield: 66%).

¹H NMR (500 MHz, CDCl₃) δ 7.59 (d, *J* = 8.2 Hz, 2H), 7.23 (d, *J* = 8.0 Hz, 2H), 7.10 (d, *J* = 8.1 Hz, 2H), 7.04 (d, *J* = 7.9 Hz, 2H), 6.30 (s, 1H), 2.71 - 2.65 (m, 1H), 2.41 - 2.32 (m, 2H), 2.30 (s, 3H), 2.25 (s, 3H), 2.21 - 2.14 (m, 1H), 2.05 (s, 3H).

### (4-11) Preparation of Compound Ed

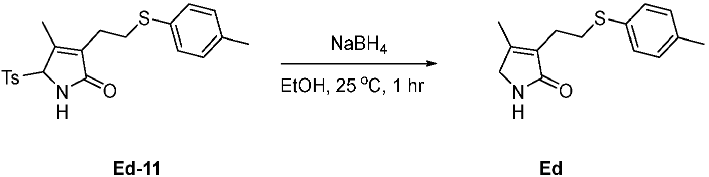

To a mixture of Compound Ed-11 (0.55 g, 1.37 mmol, 1.0 equiv.) in EtOH (50 mL), NaBH₄ (67 mg, 1.78 mmol, 1.3 equiv.) was added at 0 °C and stirred at 25 °C for 1 hour. NH₄Cl aqueous solution (30 mL) was added to the mixture, followed by extraction with DCM (60 mL × 2). The mixed organic layer was washed with brine (100 mL), dried over anhydrous Na₂SO₄, filtered and concentrated under reduced pressure condition. The residue was solidified using DCM/Hexanes to yield Compound Ed corresponding to the compound represented by Formula 4 herein in the form of white solid (305 mg, 1.23 mmol, yield: 90%).

¹H NMR (500 MHz, DMSO-*d*₆) δ 7.92 (s, 1H), 7.25 (d, *J* = 7.9 Hz, 2H), 7.13 (d, *J* = 7.9 Hz, 2H), 3.70 (s, 2H), 3.01 (t, *J* = 7.2 Hz, 2H), 2.40 (t, *J* = 6.8 Hz, 2H), 2.26 (s, 3H), 1.86 (s, 3H).
C₁₄H₁₇NOS m/z [M+H]⁺ = 248

### 1.2. Preparation of compound represented by Formula 5

Compounds H-2 and H corresponding to the compound represented by Formula 5 herein were prepared as follows (Examples 5 and 6).

### Example 5: Preparation of Compound H-2

### (5-1) Preparation of Compound H-1

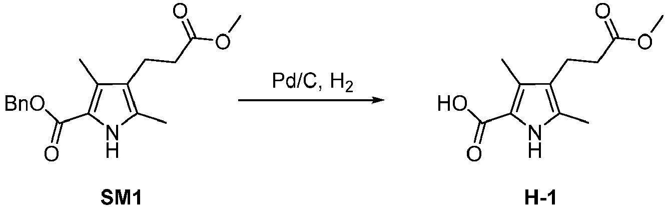

To a mixture of compound SM1 (2.5 g, 7.92 mmol, 1.0 equiv.) in THF (37.5 mL), Pd/C (1.87 g, 0.79 mmol 0.1 equiv.) was added under nitrogen condition. The mixture was degassed under vacuum condition and filled with hydrogen gas. The mixture was stirred at 25 °C for 3 hours under H₂ (15 psi) condition. Then, Pd/C was removed from the mixture under reduced pressure condition. The filtrate was concentrated under reduced pressure condition to yield Compound H-1 as a pink solid (1.57 g, 6.97 mmol, yield: 88%).

¹H NMR (400 MHz, DMSO-*d*₆) δ 11.81 (brs, 1H), 10.93 (s, 1H), 3.56 (s, 3H), 2.56 (t, *J* = 7.2 Hz, 2H), 2.35 (t, *J* = 7.7 Hz, 2H), 2.14 (s, 3H), 2.09 (s, 3H).

### (5-2) Preparation of Compound H-2

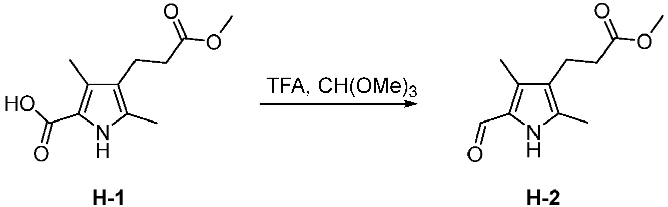

To a mixture of CH(OMe)₃ (1.93 mL, 24.86 mmol, 5.6 equiv.) and Compound H-1 (1.0 g, 4.44 mmol, 1.0 equiv.), TFA (8 mL) was added dropwise at -5 °C. The mixture was stirred at -5 °C for 1 hour. The reaction was terminated by adding NaHCO₃ aqueous solution, and the pH was adjusted to 7. After extraction with DCM (100 mL), the mixed organic layer was washed with brine (100 mL), dried over anhydrous Na₂SO₄ and concentrated under reduced pressure condition to yield Compound H-2 as a brown solid (0.89 mg, 4.26 mmol, yield : 96%).

¹H NMR (400 MHz, DMSO-*d*₆) δ 11.42 (s, 1H), 9.41 (s, 1H), 3.57 (s, 3H), 2.59 (t, *J* = 7.6 Hz, 2H), 2.40 (t, *J* = 7.7 Hz, 2H), 2.19 (s, 3H), 2.15 (s, 3H).
C₁₁H₁₅NO₃ m/z [M+H]⁺ = 210

### Example 6: Preparation of Compound H

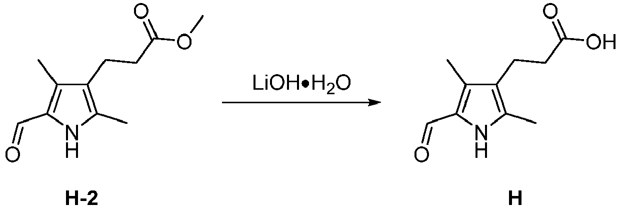

To a mixture of Compound H-2 (480 mg, 2.29 mmol, 1.0 equiv.) in methanol (7.2 mL), water (3.6 mL) and DCM (4.8 mL), lithium hydroxide (LiOH H₂O) (183 mg, 4.35 mmol, 1.9 equiv.) was added. The mixture was stirred at 35 °C for 3 hours. The mixture was neutralized with 1 M HCl aqueous solution (5 mL) and extracted with DCM (20 mL x 2). Thereafter, the mixed organic layer was washed with brine (30 mL), dried over anhydrous Na₂SO₄ and concentrated under reduced pressure condition to yield Compound H as a brown solid (0.44 mg, 2.24 mmol, yield: 98%).

¹H NMR (400 MHz, DMSO-*d*₆) δ 12.06 (s, 1H), 11.41 (s, 1H), 9.41 (s, 1H), 2.55 (t, *J* = 7.6 Hz, 2H), 2.30 (t, *J* = 7.9 Hz, 2H), 2.20 (s, 3H), 2.15 (s, 3H).
C₁₀H₁₃NO₃ m/z [M+H]⁺ = 196

### 1.3. Preparation of compound represented by Formula 1

Compounds H-Ea, E-Ea-2a, H-2-Ea, H-2-Ea-2a and H-2-Ed corresponding to the compound represented by Formula 1 herein were prepared by coupling the compound represented by Formula 4 with the compound represented by Formula 5 as follows (Examples 7 to 11).

### Example 7: Preparation of Compound H-Ea by coupling Compound H and Compound Ea

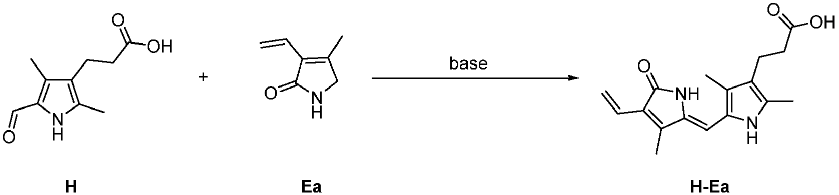

To a mixture of Compound H (100 mg, 0.51 mmol, 1.0 equiv.) in 1,4-dioxane (3 mL), piperidine (0.15 mL, 1.53 mmol, 3.0 equiv.) and Compound Ea (75.7 mg, 0.61 mmol, 1.2 equiv.) were added and stirred at 80 °C for 16 hours. After adding CHCl₃ (3 mL) to the mixture at 25 °C, the mixture was filtered under reduced pressure condition to yield a light brown solid Compound H-Ea (75 mg, 0.24 mmol, yield: 48%).

¹H NMR (500 MHz, DMSO-*d*₆) δ 10.42 (s, 1H), 6.57 (dd, *J* = 17.5, 11.5 Hz, 1H), 6.19 (dd, *J* = 17.6, 2.9 Hz, 1H), 6.07 (s, 1H), 5.28 (dd, *J* = 11.6, 2.8 Hz, 1H), 2.57 - 2.51 (m, 2H), 2.24 - 2.19 (m, 2H), 2.19 (s, 3H), 2.15 (s, 3H), 2.05 (s, 3H).

### Example 8: Preparation of Compound H-Ea-2a by coupling Compound H and Compound Ea-2a

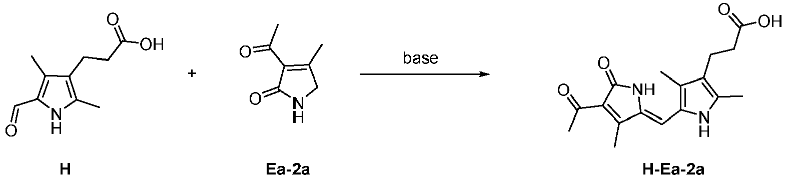

To a mixture of Compound H (20 mg, 0.10 mmol, 1.0 equiv.) in 1,4-dioxane (0.6 mL), azepane (44 µL, 0.40 mmol, 4.0 equiv.) and Compound Ea-2a (25 mg, 0.18 mmol, 1.8 equiv.) were added and stirred at 60 °C for 16 hours. After adding CHCl₃ (20 mL) to the mixture at 25 °C, the mixture was washed with 0.1 M HCl aqueous solution (15 mL × 2). The separated organic layer was dried over anhydrous Na₂SO₄, filtered and concentrated under reduced pressure condition to yield a red solid Compound H-Ea-2a (7.9 mg, 0.025 mmol, yield: 25%).
C₁₇H₂₀N₂O₄ m/z [M+H]⁺ = 317

### Example 9: Preparation of Compound H-2-Ea by coupling Compound H-2 and Compound Ea

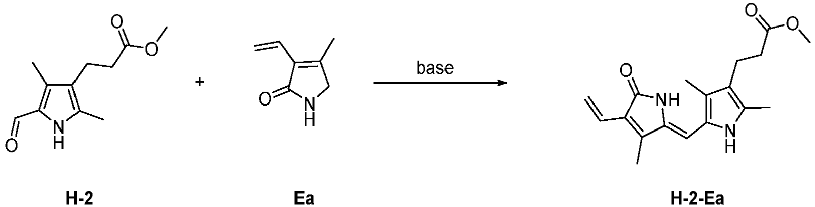

To a mixture of Compound H-2 (150 mg, 0.71 mmol, 1.0 equiv.) in 1,4-dioxane (9 mL), piperidine (0.28 mL, 2.86 mmol, 4.0 equiv.) and Compound Ea (75.7 mg, 1.28 mmol, 1.8 equiv.) were added and stirred at 60 °C for 16 hours. After adding CHCl₃ (100 mL) to the mixture at 25 °C, the mixture was washed with 0.1 M HCl aqueous solution (80 mL x 2). The separated organic layer was dried over anhydrous Na₂SO₄, filtered and concentrated under reduced pressure condition to yield Compound H-2-Ea as a brown solid (70 mg, 0.22 mmol, yield: 31%).

¹H NMR (500 MHz, DMSO-d₆) δ 10.41 (s, 1H), 9.88 (s, 1H), 6.57 (dd, *J* = 17.5, 11.5 Hz, 1H), 6.20 (dd, *J* = 17.6, 2.8 Hz, 1H), 6.07 (s, 1H), 5.29 (dd, *J* = 11.6, 2.8 Hz, 1H), 3.57 (s, 3H), 2.59 (t, *J=* 7.6 Hz, 2H), 2.39 (t, *J=* 7.6 Hz, 2H), 2.18 (s, 3H), 2.15 (s, 3H), 2.05 (s, 3H).

### Example 10: Preparation of Compound H-2-Ea by coupling Compound H-2 and Compound Ea-2a

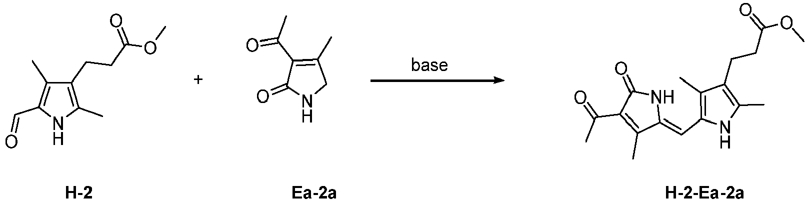

To a mixture of Compound H-2 (150 mg, 0.71 mmol, 1.0 equiv.) in 1,4-dioxane (9 mL), piperidine (0.32 mL, 3.2 mmol, 4.5 equiv.) and Compound Ea-2a (157 mg, 1.3 mmol, 1.8 equiv.) were added and stirred at 60 °C for 16 hours. After adding CHCl₃ (100 mL) to the mixture at 25 °C, the mixture was washed with 0.1 M HCl aqueous solution (80 mL x 2). The separated organic layer was dried over anhydrous Na₂SO₄, filtered and concentrated under reduced pressure condition to yield Compound H-2-Ea-2a as a reddish-brown solid (160 mg, 0.50 mmol, yield: 71%).

¹H NMR (400 MHz, DMSO-d₆) δ 10.72 (s, 1H), 10.09 (s, 1H), 6.43 (s, 1H), 3.57 (s, 3H), 2.61 (m, 2H), 2.40 (s, 3H), 2.39 (s, 3H), 2.38 (m, 2H), 2.23 (s, 3H), 2.11 (s, 3H).

### Example 11: Preparation of Compound H-2-Ed by coupling Compound H-2 and Compound Ed

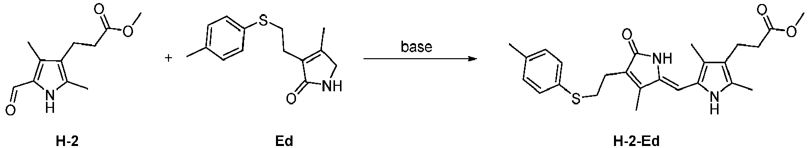

To a mixture of Compound H-2 (100 mg, 0.48 mmol, 1.0 equiv.) in 1,4-dioxane (3 mL), azepane (0.16 mL, 1.4 mmol, 3.0 equiv.) and Compound Ed (142 mg, 0.58 mmol, 1.2 equiv.) were added and stirred at 80 °C for 16 hours. After adding CHCl₃ (40 mL) to the mixture at 25 °C, the mixture was washed with 0.1 M HCl aqueous solution (30 mL x 2). The separated organic layer was dried over anhydrous Na₂SO₄, filtered and concentrated under reduced pressure condition to yield Compound H-2-Ed as a yellow solid (165 mg, 0.37 mmol, yield: 78%).

¹H NMR(500 MHz, DMSO-d₆) δ 10.30 (s, 1H), 9.79 (s, 1H), 7.26 (d, J= 7.7 Hz, 2H), 7.14 (d, *J* = 7.8 Hz, 2H), 5.94 (s, 1H), 3.56 (s, 3H), 3.05 (t, *J* = 7.7 Hz, 2H), 2.57 (t, *J* = 7.5 Hz, 2H), 2.55 (overlapped with DMSO-*d*₆'s signal, 2H), 2.37 (t, *J* = 7.9 Hz, 2H), 2.26 (s, 3H), 2.16 (s, 3H), 2.02 (s, 3H).

### 2. Preparation of compound represented by Formula 2 from compound represented by Formula 1

The compound represented by Formula 1 prepared above was dimerized to prepare compounds corresponding to the compound represented by Formula 2 herein, and then bilirubin (F-3a) was prepared therefrom.

### Example 12: Preparation of Compound C-Ed by dimerization of Compound H-2-Ed

### (12-1) Preparation of C-Ed-v by dimerization of H-2-Ed

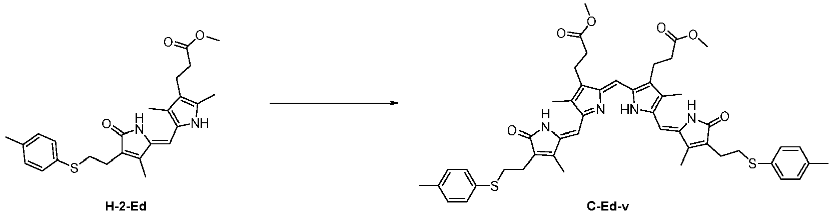

To a mixture of Compound H-2-Ed (32.0 mg, 0.073 mmol, 1.0 equiv.) in DCM (15 mL),*p*-chloranil (45 mg, 0.18 mol, 2.5 equiv.) and formic acid (0.73 mL, 0.1M) were added and stirred at 50 °C for one day (It can be confirmed that the mixture turns green). After adding DCM (30 mL) to the mixture, it is washed sequentially with NaHCO₃ aqueous solution (10 mL x 2) and 4% NaOH aqueous solution (10 mL x 2). The mixed organic layer was washed with water (20 mL x 2), dried over anhydrous Na₂SO₄ and concentrated to yield green Compound C-Ed-v (60 mg, 0.069 mmol, yield: 95%).
C₄₉H₅₄N₄O₆S₂ m/z [M+H]⁺ = 860

### (12-2) Preparation of C-Ed from C-Ed-v

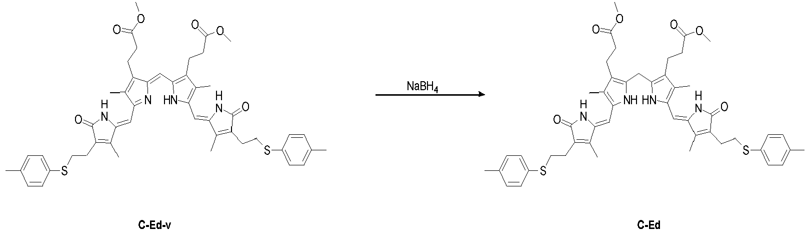

To a mixture of Compound C-Ed-v (60 mg, 0.073 mmol, 1.0 equiv.) in chloroform (11 mL) and methanol (11 mL), NaBH₄ (155 mg, 4.14 mmol, 60.0 equiv.) was added at 0 °C and then stirred at 0 °C for 10 minutes. Chloroform (20 mL) was added, and this solution was washed with NH₄Cl aqueous solution (20 mL x 2). The mixed organic layer was dried over anhydrous Na₂SO₄, filtered and concentrated under reduced pressure condition to yield Compound C-Ed (50 mg, 0.058 mmol, yield: 79%)
C₄₉H₅₆N₄O₆S₂ m/z [M+H]⁺ = 862

### Example 13: Preparation of Compound F-3a from Compound C-Ed

### (13-1) Preparation of D-Ed from C-Ed

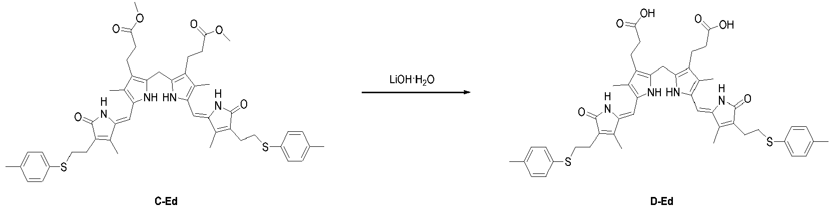

To a mixture of Compound C-Ed (50 mg, 0.058 mmol, 1.0 equiv.) in methanol (5 mL), a mixture of LiOH·H₂O (14.62 mg, 0.34 mmol, 6.0 equiv.) in water (1 mL) was added and stirred at 60 °C for 2 hours under nitrogen condition. CHCl₃ (30 mL) was added to the mixture and the pH of the mixture was adjusted to 3 with 0.1 M HCl aqueous solution (8 mL). The mixed organic layer was dried over anhydrous Na₂SO₄, filtered and concentrated under reduced pressure condition. Methanol (10 mL) was added to the residue and the precipitated solid was filtered under reduced pressure to yield Compound D-Ed as an orange solid (20 mg, 0.024 mmol, yield: 41%).
C₄₇H₅₂N₄O₆S₂ m/z [M+H]⁺ = 833

### (13-2) Preparation of F-3a from D-Ed

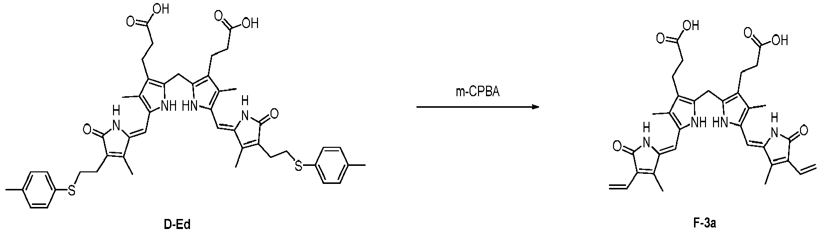

To a mixture of Compound D-Ed (15 mg, 0.018 mmol, 1.0 equiv.) in toluene (1 mL), m-CPBA (3.41 mg, 0.020 mmol, 1.1 equiv.) was added at 0 °C. The mixture was stirred while refluxing for 30 minutes. After completion of the reaction, the reaction was terminated with an aqueous solution of NaHSO₃. Water (100 mL) was added to the mixture then extracted with CHCl₃ (100 mL x 2). The separated organic layer was dried over anhydrous Na₂SO₄ and filtered. The filtered solution was concentrated under reduced pressure condition, and the residue was triturated with a MeOH:DCM (1:1) solution, followed by filtration to yield an orange Compound F-3a.
C₃₃H₃₆N₄O₆ m/z [M+H]⁺ = 585

### 3. Preparation of compound represented by Formula 3 (Claims 4 and 8)

Compound B corresponding to the compound represented by Formula 9 was prepared as follows, and then compounds C and D corresponding to Formula 8 were prepared therefrom. Thereafter, the compound corresponding to Formula 3 were prepared by coupling the above compound with the compound corresponding to Formula 4.

### 3.1. Preparation of compound represented by Formula 9

### Example 14: Preparation of Compound B

### (14-1) Preparation of Compound A

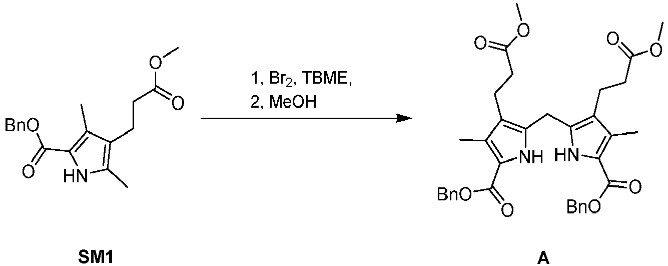

A mixture of Br₂ (53.2 g, 333 mmol, 1.4 equiv.) in MTBE (375 mL) was added dropwise to a mixture of compound SM1 (75.0 g, 238 mmol, 1.0 equiv.) in MTBE (1125 mL) at 20 °C under nitrogen condition. The mixture was stirred at 20 °C for 1 hour, followed by confirming complete reaction through TLC. The solvent was removed under reduced pressure condition. Then, methanol (546 mL) was added to the mixture. The mixture was stirred at 50 °C for 12 hours, followed by confirming complete consumption of the reactant through TLC. The mixture was cooled to 20 °C and concentrated under reduced pressure condition. After the mixture was triturated with methanol (100 mL) at 20 °C, the filtered product was washed with methanol (50 mL x 2) to yield Compound A as a gray solid (59.0 g, 95.9 mmol, yield: 81%).

¹H NMR (400 MHz, CDCl₃) δ 9.11 (s, 2H), 7.41 - 7.25 (m, 10H), 5.26 (s, 4H), 3.97 (s, 2H), 3.58 (s, 6H), 2.77 (t, *J=* 7.2 Hz, 4H), 2.52 (t, *J=* 6.8 Hz, 4H), 2.29 (s, 6H).

### (14-2) Preparation of Compound B

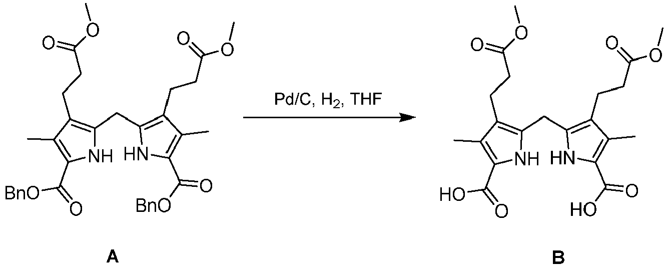

To a mixture of Compound A (50.0 g, 81.3 mmol, 1.0 equiv.) prepared above in THF (650 mL), Pd/C (5.00 g, 10 mol%) was added under nitrogen condition. The mixture was degassed under vacuum conditions and filled with H₂. The mixture was stirred at 20 °C for 16 hours under H₂ condition (15 psi). Then, a mixture of Na₂CO₃ (8.62 g, 81.3 mmol) and H₂O (50 mL) was added to the mixture and stirred for 0.5 hours. The mixture was filtered and the filtrate was adjusted to pH 7 by the addition of acetic acid (ca. 10 mL). The precipitate was filtered and dried to yield Compound B corresponding to the compound represented by Formula 9 herein as a pink solid (34.0 g, 78.3 mmol, yield: 96%).

¹H NMR (400 MHz, DMSO-d₆) δ 11.09 (s, 2H), 3.78 (s, 2H), 3.56 (s, 6H), 2.56 (t, *J* = 7.2 Hz, 4H), 2.16 - 2.10 (m, 10H).

### 3.2. Preparation of compound represented by Formula 8

### Example 15: Preparation of Compound C

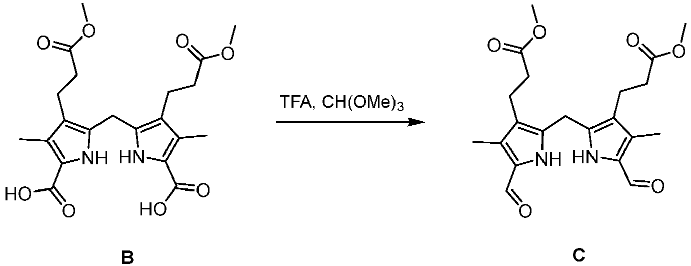

Compound B (20.0 g, 46.1 mmol, 1.0 equiv.) prepared above was added to TFA (190 mL) at 0 °C. The mixture was stirred at 0 °C for 1 hour under nitrogen condition, and trimethoxymethane (55.2 g, 520 mmol, 11.3 equiv.) was added to the mixture at 0 °C. Thereafter, the mixture was stirred at 0 °C for 1 hour and monitored by LCMS. 1.7 L of water was added to the mixture and the mixture was stirred for 10 minutes. The precipitate was filtered and washed with 0.3 L of water. The raw material was triturated with ethanol (0.2 L) and ammonium hydroxide (0.4 L) at 20 °C for 30 minutes. The yellow precipitate was filtered and then washed with water (0.3 L). Methanol (0.4 L) was added to the mixture and refluxed for 10 minutes. After cooling the mixture at room temperature, the precipitate was filtered and washed with methanol (0.1 L) at 5 °C thus to yield Compound C corresponding to the compound represented by Formula 8 herein as a brown solid (12.0 g, 29.8 mmol, yield: 65%).

¹H NMR (400 MHz, CDCl₃) δ 10.19 - 10.00 (m, 2H), 9.47 (s, 2H), 4.05 (s, 2H), 3.71 (s, 6H), 2.80 (t, *J* = 7.2 Hz, 4H), 2.61 - 2.45 (m, 4H), 2.29 (s, 6H).

### Example 16: Preparation of Compound D

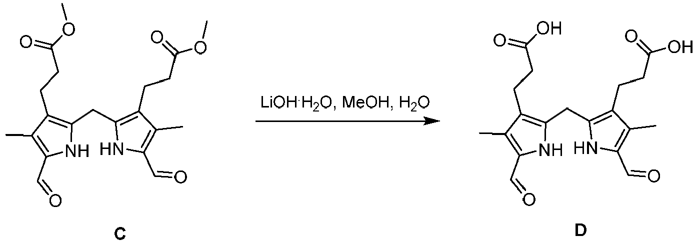

Lithium hydroxide (LiOH·H₂O) (2.75 g, 65.6 mmol, 6.6 equiv.) was added to a mixture of methanol (100 mL) and water (100 mL) as well as Compound C (4.00 g, 9.94 mmol, 1.0 equiv.) of Example 15 above. The mixture was stirred at 25 °C for 16 hours, and the residue was diluted with 100 mL of water. 1M hydrochloric acid was added dropwise to the mixture to adjust the pH to 2-3. Then, the precipitate was filtered and dried to yield Compound D corresponding to the compound represented by Formula 8 herein as a purple solid (3.49 g, 9.32 mmol, yield: 94%).

¹H NMR (400 MHz, CDCl₃) δ 12.03 (brs, 2H), 11.51 (s, 2H), 9.48 (s, 2H), 3.91 (s, 2H), 2.54 (overlapped with DMSO-*d*₆'s signal, 4H), 2.18 (s, 6H), 2.06 (t, *J* = 8.0 Hz, 4H).
C₁₉H₂₂N₂OS m/z [M+H]⁺ = 375

### 3.3. Preparation of compound represented by Formula 3

### Example 17: Preparation of Compound Ds-Ea by coupling Compound D and Compound Ea

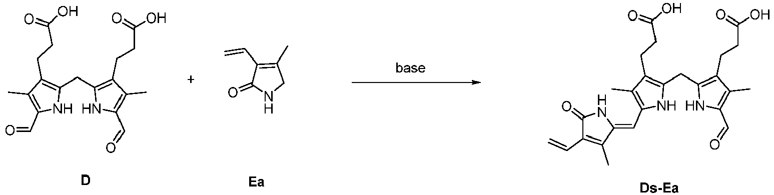

To a mixture of Compound D (1.10 g, 2.94 mmol, 1.0 equiv.) in 1,4-dioxane (30 mL) and DMSO (10 mL), piperidine (2.0 g, 23.5 mmol, 8.0 equiv.) and Compound Ea (362 mg, 2.94 mmol, 1.0 equiv.) were added. This mixture was stirred at 25 °C for 30 hours under nitrogen condition. The mixture was concentrated under reduced pressure condition. The residue was purified by prep-HPLC to yield Compound Ds-Ea as a yellow solid (125 mg, 0.26 mmol, yield: 9%).

¹H NMR (400 MHz, DMSO-d₆) δ 11.95 (brs, 1H), 11.38 (s, 1H), 10.46 (s, 1H), 9.95 (s, 1H), 9.46 (s, 1H), 6.58 (dd, *J* = 17.6, 11.6 Hz, 1H), 6.22 (dd, *J* = 17.6, 2.8 Hz, 1H), 6.09 (1s, 1H), 5.30 (dd, *J=* 2.8 Hz, 1H), 3.95 (s, 2H), 2.55 - 2.52 (m, 2H), 2.47 - 2.43 (m, 2H), 2.18 (d, *J=* 11.2 Hz, 6H), 2.08 (t, *J* = 8.0 Hz, 2H), 2.02 (s, 3H), 1.98 (t, *J* = 8.0 Hz, 2H)

### Example 18: Preparation of Compound Ds-Ga by coupling Compound D and Compound Ea

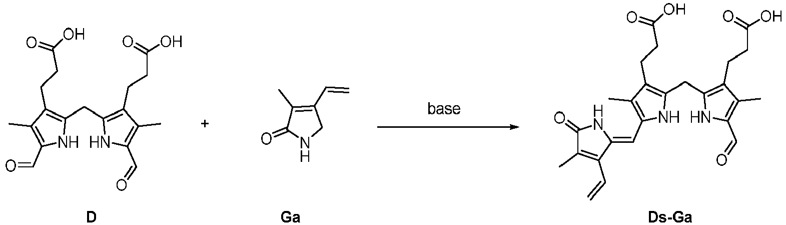

To a mixture of Compound D (608 mg, 1.62 mmol, 1.0 equiv.) and Compound Ga (200 mg, 1.62 mmol, 1.0 equiv.) in 1,4-dioxane (20 mL), piperidine (1.11 g, 12.99 mmol, 8.0 equiv.) was added. The mixture was stirred at 25 °C for 16 hours. The mixture was concentrated under reduced pressure condition. The residue was purified by prep-HPLC to yield Compound Ds-Ga as a red solid (35 mg, 0.073 mmol, yield: 4%).

¹H NMR (400 MHz, DMSO-d₆) δ 11.96 (brs, 1H), 11.36 (s, 1H), 10.38 (s, 1H), 10.02 (s, 1H), 9.47 (s, 1H), 6.79-6.86 (m, 1H), 6.09 (s, 1H), 5.67 - 5.64 (m, 1H), 5.63 - 5.61 (m, 1H), 3.94 (s, 2H), 2.57 - 2.53 (m, 2H), 2.45 - 2.43 (m, 2H), 2.19 (s, 3H), 2.11 - 2.06 (m, 2H), 1.99 (s, 3H), 1.98 - 1.96 (m, 2H), 1.93 (s, 3H).

### 4. Preparation of compound represented by Formula 2 from compound represented by Formula 3

Compound F-9a corresponding to the compound represented by Formula 2 herein was prepared by coupling compounds Ds-Ea and Ds-Ga represented by Formula 3 with compounds Ga and Ea corresponding to the compound represented by Formula 4 herein, respectively.

### Example 19: Preparation of Compound F-9a by coupling Compound Ds-Ea and Compound Ga

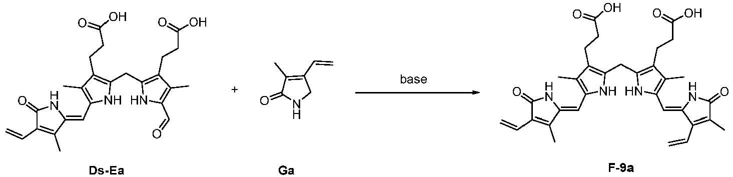

To a mixture of Compound Ds-Ea (173 mg, 0.360 mmol, 1.0 equiv.) and 1,4-dioxane (5 mL), piperidine (307 mg, 3.60 mmol, 0.356 mL, 10.0 equiv.) and Compound Ga (133 mg, 1.08 mmol, 3.0 equiv.) were added, and the mixture was stirred at 100 °C for 12 hours under nitrogen condition. After adding DCM (200 mL) to the mixture, it was washed with 0.2 M aqueous hydrochloric acid (50 mL x 2). The organic layer was separated, dried over anhydrous Na₂SO₄, filtered and concentrated under reduced pressure condition. The residue was triturated with methanol (50 L). The red solid was filtered and washed with methanol (10 mL x 2) to yield Compound F-9a as a red solid (141 mg, 0.241 mmol, yield: 67%).

¹H NMR (400 MHz, DMSO-*d₆*) δ 11.91 (brs, 2H), 10.49 (s, 1H), 10.46 (s, 1H), 10.05 (s, 1H), 9.92 (s, 1H), 6.87 - 6.79 (m, 1H), 6.62 - 6.56 (m, 1H), 6.24 - 6.19 (m, 1H), 6.10 (s, 2H), 5.66 - 5.61 (m, 2H), 5.32 - 5.29 (m, 1H), 3.99 (s, 2H), 2.43 (t, *J=* 7.6 Hz, 4H), 2.17 (s, 3H), 2.04 (s, 3H), 2.00 (s, 3H), 1.96 (t, *J=* 7.6 Hz, 4H), 1.93 (s, 3H).
C₃₃H₃₆N₄O₆ m/z [M+H]⁺ = 585.3

### Example 20: Preparation of Compound F-9a by coupling Compound Ds-Ga and Compound Ea

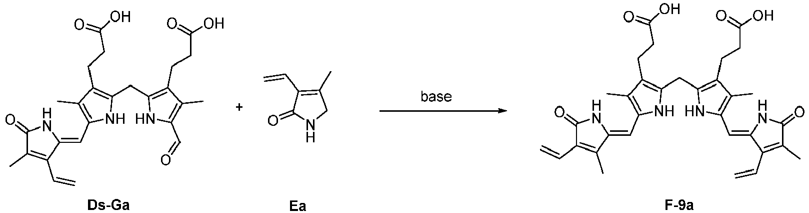

Piperidine (76 mg, 0.90 mmol, 10.0 equiv.) and Compound Ea (33 mg, 0.27 mmol, 3.0 equiv.) were added to a mixture of Compound Ds-Ga (43 mg, 0.090 mmol, 1.0 equiv.) and 1,4-dioxane (2 mL), and the mixture was stirred at 100 °C for 12 hours under nitrogen condition. After removing the solvent under reduced pressure condition, CHCl₃ (100 mL) was added to the residue and washed with 0.1M aqueous hydrochloric acid (30 mL x 2). The organic layer was separated, dried over anhydrous Na₂SO₄, filtered and concentrated under reduced pressure condition. The residue was triturated with methanol (10 mL) at 20 °C. The filtered red solid was washed with methanol (10 mL x 2) to yield Compound F-9a as a red solid (10 mg, 0.017 mmol, yield: 19%).

¹H NMR (400 MHz, DMSO-d₆) δ 11.89 (brs, 2H), 10.49 (s, 1H), 10.46 (s, 1H), 10.05 (s, 1H), 9.92 (s, 1H), 6.86 - 6.78 (m, 1H), 6.62 - 6.54 (m, 1H), 6.23 - 6.18 (m, 1H), 6.09 (s, 2H), 5.65 - 5.61 (m, 2H), 5.31 - 5.28 (m, 1H), 3.98 (s, 2H), 2.42 (t, *J* = 7.6 Hz, 4H), 2.16 (s, 3H), 2.03 (s, 3H), 2.00 (s, 3H), 1.95 (t, *J=* 7.6 Hz, 4H), 1.92 (s, 3H).
C₃₃H₃₆N₄O₆ m/z [M+H]⁺ = 585.3

### 5. PEGylation of compound represented by Formula 2

Compound F-9a corresponding to the compound represented by Formula 2 prepared above was PEGylated to prepare Compound FP-9a-1.

### Example 21: PEGylation of Compound F-9a

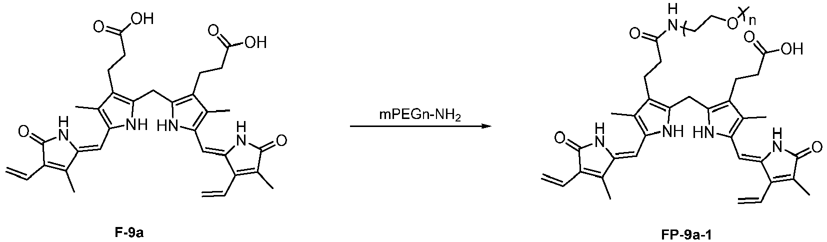

Compound F-9a (500 mg, 0.85 mmol, 1.0 equiv.) was dissolved in DMSO (40 mL) and stirred at 25 °C for 15 minutes. To this mixture, a mixture of CDI (210 mg, 1.30 mmol, 1.5 equiv.) in DMSO (10 mL) was added dropwise. After stirring at 25 °C for 2 hours, a mixture of mPEG₃₆-NH₂ (553 mg, 0.34 mmol, 0.4 equiv.) in DMSO (5 mL) was added and stirred at 25 °C for 4 hours. The mixture was dissolved in aqueous Na₂CO₃ solution (250 mL) and stirred until it became a clear yellow solution. After extraction with CHCl₃ (200 mL x 3) and drying the mixed organic layer over anhydrous MgSO₄, it was filtered and concentrated under reduced pressure condition. After precipitation by adding MTBE to the filtrate, filtration was performed to yield a yellow solid Compound FP-9a-1 (360 mg, 0.16 mmol, yield: 48%).

## Claims

1. A method for synthesizing bilirubin, comprising preparing a compound represented by Formula 2 below by dimerizing a compound represented by Formula 1 below: 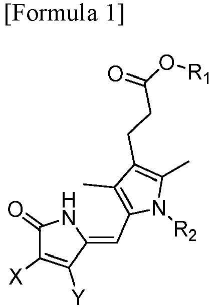 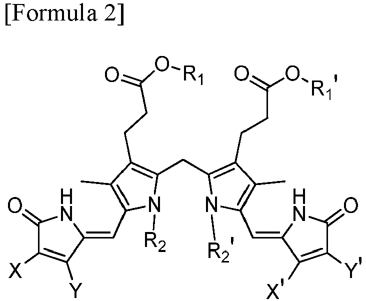 (wherein, in the above formulas 1 and 2, R₁ and R₁' are each independently hydrogen, an alkyl group having 1 to 12 carbon atoms, an aryl group having 6 to 20 carbon atoms, a heteroaryl group having 2 to 20 carbon atoms, an arylalkyl group having 7 to 20 carbon atoms, or a heteroarylalkyl group having 3 to 20 carbon atoms; R₂ and R₂' are each hydrogen or a nitrogen protecting group; any one of X and Y is a vinyl or acetyl group, or a halogen atom, or an ethyl group substituted with a hydroxyl group, sulfide, selenide or a halogen atom while the other is a methyl group; and any one of X' and Y' is a vinyl or acetyl group, or a halogen atom, or an ethyl group substituted with a hydroxyl group, sulfide, selenide or a halogen atom while the other is a methyl group).

2. The method according to claim 1, further comprising reacting the compound represented by Formula 2 with polyethylene glycol (PEG).

3. The method according to claim 1, wherein the compound represented by Formula 1 is reacted with polyethylene glycol (PEG) and then dimerized.

4. A compound represented by Formula 3 below: 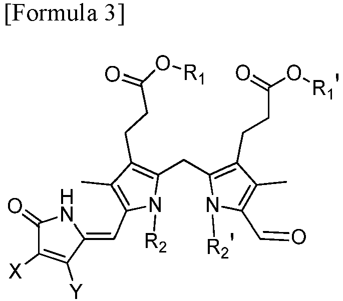 (wherein, R₁ and R₁' are each independently hydrogen, an alkyl group having 1 to 12 carbon atoms, an aryl group having 6 to 20 carbon atoms, a heteroaryl group having 2 to 20 carbon atoms, an arylalkyl group having 7 to 20 carbon atoms, or a heteroarylalkyl group having 3 to 20 carbon atoms; R₂ and R₂' are each hydrogen or a nitrogen protecting group; and any one of X and Y is a vinyl or acetyl group, or a halogen atom, or an ethyl group substituted with a hydroxyl group, sulfide, selenide or a halogen atom while the other is a methyl group).

5. A method for synthesizing bilirubin, comprising preparing a compound represented by Formula 2 below by coupling a compound represented by Formula 4 below with the compound represented by Formula 3 of claim 4: 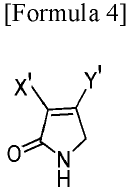 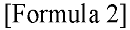 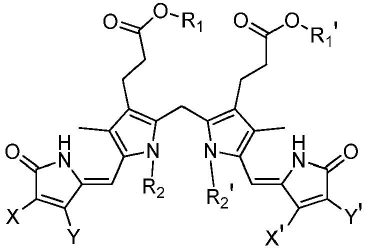 (wherein, in the formulas 4 and 2, R₁ and R₁' are each independently hydrogen, an alkyl group having 1 to 12 carbon atoms, an aryl group having 6 to 20 carbon atoms, a heteroaryl group having 2 to 20 carbon atoms, an arylalkyl group having 7 to 20 carbon atoms, or a heteroarylalkyl group having 3 to 20 carbon atoms; R₂ and R₂' are each hydrogen or a nitrogen protecting group; any one of X and Y is a vinyl or acetyl group, or a halogen atom, or an ethyl group substituted with a hydroxyl group, sulfide, selenide or a halogen atom while the other is a methyl group; and any one of X' and Y' is a vinyl or acetyl group, or a halogen atom, or an ethyl group substituted with a hydroxyl group, sulfide, selenide or a halogen atom while the other is a methyl group).

6. The method according to claim 5, further comprising reacting the compound represented by Formula 2 with polyethylene glycol (PEG).

7. The method according to claim 5, wherein the compound represented by Formula 3 is reacted with polyethylene glycol (PEG) and then coupled with the compound represented by Formula 4.

8. The method according to claim 5, further comprising reacting a compound represented by Formula 1 below with a compound represented by Formula 5 below to prepare the compound represented by Formula 3: 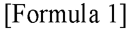 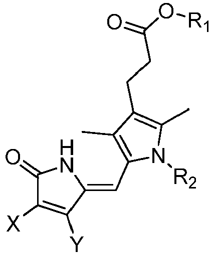 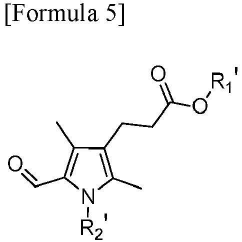 (wherein, R₁, R₁', R₂, R₂', X and Y in Formulas 1 and 5 are the same as R₁, R₁', R₂, R₂', X and Y in Formula 3, respectively).

9. The method according to claim 5, further comprising substituting a carboxyl group bonded to a pyrrole group of a compound represented by Formula 6 below with an aldehyde group to prepare the compound represented by Formula 3: 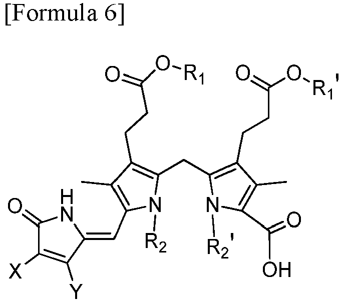 (wherein, R₁, R₁', R₂, R₂', X and Y are the same as R₁, R₁', R₂, R₂', X and Y in Formula 3, respectively).

10. The method according to claim 9, further comprising coupling a compound represented by Formula 7 below with the compound represented by Formula 4 of claim 5 to prepare the compound represented by Formula 6: 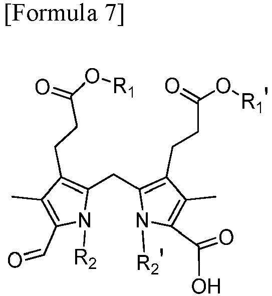 (wherein, R₁, R₁', R₂ and R₂' are the same as R₁, R₁', R₂ and R₂' in Formula 6, respectively).

11. The method according to claim 5, further comprising coupling a compound represented by Formula 8 below with the compound represented by Formula 4 to prepare the compound represented by Formula 3: 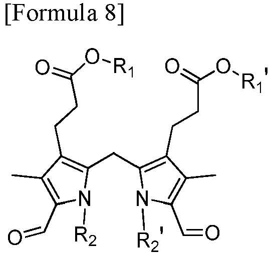 (wherein, R₁, R₁', R₂ and R₂' are the same as R₁, R₁', R₂ and R₂' in Formula 3, respectively).

12. The method according to claim 10, further comprising substituting any one carboxyl group bonded to a pyrrole group of a compound represented by Formula 9 below with an aldehyde group to prepare the compound represented by Formula 7: 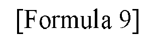 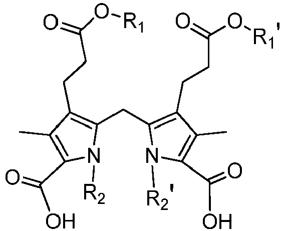 (wherein, R₁, R₁', R₂ and R₂' are the same as R₁, R₁', R₂ and R₂' in Formula 7, respectively).

13. The method according to claim 11, further comprising substituting a carboxyl group bonded to a pyrrole group of a compound represented by Formula 7 or 9 below with an aldehyde group to prepare the compound represented by Formula 8: 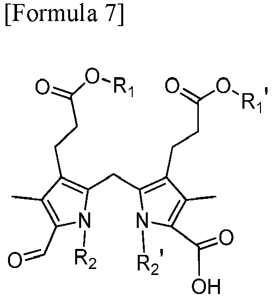 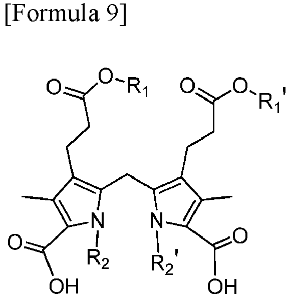 (wherein, in the Formulas 7 and 9, R₁, R₁', R₂ and R₂' are the same as R₁, R₁', R₂ and R₂' in Formula 8, respectively).
